(19) Europäisches Patentamt
European Patent Office
Office européen des brevets

(11) **EP 4 571 308 A1**

(12) **EUROPÄISCHE PATENTANMELDUNG**

(43) Veröffentlichungstag:
**18.06.2025** Patentblatt 2025/25

(21) Anmeldenummer: 23216372.5

(22) Anmeldetag: **13.12.2023**

(51) Internationale Patentklassifikation (IPC):
*G01N 33/50* (2006.01)    *C12M 1/12* (2006.01)
*C12M 1/00* (2006.01)    *C12M 1/34* (2006.01)
*G01N 21/77* (2006.01)    *G01N 33/58* (2006.01)

(52) Gemeinsame Patentklassifikation (CPC):
**G01N 33/5005; C12M 25/04; C12M 25/14;
C12M 29/00; C12M 41/26; C12M 41/34;
G01N 21/77; G01N 33/582;** G01N 21/6408;
G01N 21/6458; G01N 2021/7786; G01N 2021/7796

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **Karlsruher Institut für Technologie
76131 Karlsruhe (DE)**

(72) Erfinder:
• **Gottwald, Eric
76149 Karlsruhe (DE)**
• **Grün, Christoph
76829 Landau (DE)**
• **Zweigerdt, Robert
30559 Hannover (DE)**

(74) Vertreter: **Gille Hrabal Partnerschaftsgesellschaft
mbB
Patentanwälte
Brucknerstraße 20
40593 Düsseldorf (DE)**

(54) **VERFAHREN ZUR BESTIMMUNG VON SAUERSTOFFKONZENTRATIONEN IN 3D-ZELLKULTUREN VON KARDIOMYOZYTEN ODER HERZORGANOIDEN**

(57)    Die Erfindung betrifft ein Verfahren zur *in vitro*-Messung von Sauerstoffkonzentrationen und/oder Sauerstoffkonzentrationsgradienten in 3D-Zellkulturen von Kardiomyozyten oder an Herzorganoiden (HFOs) in dreidimensionalen Strukturen die aus Fluorophor-dotierten Sauerstoff-sensitiven Sensorfolien gebildet sind, optional in Kombination mit der Messung weiterer physiologisch relevanter Parameter. Die stellt damit neue physiologisch relevantere *in vitro*-Modelle zur Untersuchung von Kardiotoxizität und ischämischer Toxizität bereit.

FIG. 3

**EP 4 571 308 A1**

**Beschreibung**

Die vorliegende Erfindung betrifft ein Verfahren zur *in vitro*-Messung von Sauerstoffkonzentrationen und/oder Sauerstoffkonzentrationsgradienten in 3D-Zellkulturen von Kardiomyozyten oder Herzorganoiden in dreidimensionalen Strukturen, die aus Fluorophor-dotierten Sauerstoff-sensitiven Sensorfolien gebildet sind. Das erfindungsgemäße Verfahren ermöglicht insbesondere auch die simultane Bestimmung weiterer Parameter wie intrazellulärer Calciumkonzentrationen oder Calciumtransienten, $CO_2$- und Glukose-Konzentrationen, sowie pH-Wert in unmittelbarer Mikroumgebung der 3D-Zellkulturen. Damit stellt das erfindungsgemäße Verfahren neue physiologisch relevantere *in* vitro-Modelle zur Untersuchung von Kardiotoxizität und ischämischer Toxizität bereit.

**HINTERGRUND UND STAND DER TECHNIK**

In Zellkulturen spielen gewebetypische Analytkonzentrationen, wie z.B. die Sauerstoff-, $CO_2$- und Glukosekonzentration oder der pH-Wert eine wichtige Rolle. Informationen zum Sauerstoffgradienten bzw. die exakte Messung der Sauerstoffkonzentration in der Mikroumgebung dreidimensionaler Zellkulturen sind notwendig, um beispielsweise Aussagen über die Versorgung der Zellen treffen zu können und zu beurteilen, ob die Kulturbedingungen der physiologischen Situation entsprechen.

In der Regel werden Zellkulturen unter Umgebungsatmosphäre, also mit einer Konzentration von 21 % Sauerstoff kultiviert, die in keinem Gewebe erreicht wird und somit zu einer Sauerstoffüberversorgung (Hyperoxie) führt, was Stressbedingungen in der Zellkultur mit unphysiologischen Antworten auf äußere Reize, wie z.B. auf Chemikalien, führt. Um gewebetypische Sauerstoffkonzentrationen in Zellkulturen einstellen zu können, sind entsprechende Analyseverfahren notwendig. Sauerstoff kann hierzu beispielsweise polarographisch bestimmt werden, was jedoch wiederum mit Sauerstoffverbrauch verbunden ist und nicht für solche Anwendungen geeignet ist. Für Zellkulturen ebenso wenig geeignet ist die Winkler-Methode, bei der Sauerstoff durch Zugabe von Manganchlorid und Kaliumiodid und dem daraus entstehenden braunen Niederschlag aus Manganoxidhydroxid ermittelt wird.

Ein alternativer Ansatz der Sauerstoffmessung beruht auf der dynamischen Fluoreszenzlöschung. Hierbei wird ein Fluorophor durch Bestrahlen mit Licht einer bestimmten Wellenlänge angeregt, welches daraufhin das Fluoreszenzemissionslicht entweder abgibt oder strahlungsfrei auf ein Sauerstoffmolekül überträgt. Dadurch sinkt die Emission, wodurch die Sauerstoffkonzentration bestimmt werden kann. Solche Systeme sind weit verbreitet und werden von einigen Firmen kommerziell vertrieben. Hierbei sind die Fluorophore entweder auf eine flexible Polymerträgerfolie aufgebracht oder in Form von Hydrogelen oder in Nanopartikeln immobilisiert. Als Fluorophore werden hierfür häufig Platin-II- bzw. Palladium-II-Porphyrine oder Ruthenium-II-Komplexe verwendet [Wang, X.D. and O.S. Wolfbeis; Optical methods for sensing and imaging oxygen: materials, spectroscopies and applications. Chem. Soc. Rev. 2014, 43 (10), 3666 - 761*]*.

Für eine zweidimensionale Kultivierung von Zellen sind solche planaren Foliensysteme ausreichend. Eine zweidimensionale Anordnung von Zellen kommt jedoch, außer an der Innenwand von Blutgefäßen und in der Lunge, im menschlichen Körper nicht vor. Stattdessen erfahren (*in vivo*) Gewebe Sauerstoffgradienten, die mit der Dicke der Gewebe zunehmen bzw. durch Blutgefäße abgefedert werden. Die Bestimmung von Sauerstoffkonzentrationen sowie von Sauerstoffgradienten in unmittelbarer Mikroumgebung von 3D-Zellkulturen von Kardiomyozytenaggregaten oder Herzorganoiden ist mit oben beschriebenen Verfahren nicht möglich. Dies ist jedoch enorm wichtig für die Etablierung organotypischer Zell- und Gewebekulturen, damit eine verbesserte Übertragbarkeit solcher Versuche auf die menschliche Situation gewährleistet ist.

Daten aus *in* vitro-Experimenten lassen sich derzeit nur eingeschränkt auf die *in* vivo-Situation übertragen. Dies führt dazu, dass immer noch zahlreiche Tierversuche im Bereich der Medikamentenentwicklung durchgeführt werden müssen und selbst diese führen nicht immer zu Daten, die auf die menschliche Situation übertragen werden können. So wurden in den letzten 60 Jahren 462 zugelassene Medikamente aufgrund toxischer Nebenwirkungen wieder vom Markt genommen [Onakpoya, I. J.; Heneghan, C. J.; Aronson, J. K.; Post-marketing withdrawal of 462 medicinal products because of adverse drug reactions: a systematic review of the world literature. BMC Med. 2016, 14 (10)*]*. Es besteht daher dringender Bedarf an geeigneten Modellsystemen, die die humane Situation besser nachbilden, dazu gehören 3D-Zellkulturen bzw. Organ-on-Chips. Ein Parameter, der bisher meist vernachlässigt wurde, ist die Sauerstoffkonzentration in den Kulturmodellen. Maßgeblich ist dabei die Entwicklung eines Systems, das Sauerstoff in unmittelbarer Mikroumgebung von 3D-Zellkulturen messen kann. Dies ist notwendig, um physiologisch relevantere *in* vitro-Modelle zu generieren. Ein Großteil der heute durchgeführten *in* vitro-Experimente findet bei einer globalen Sauerstoffkonzentration von 18 bis 21% statt, also weit über den physiologischen Sauerstoffkonzentrationen, die gewebeabhängig zwischen nur 0,5 und 14% liegen [Wenger, R. H.; Kurtcuoglu, V.; Scholz, C. C.; Mart, H. H.; Hoogewijs, D.; Frequently asked questions in hypoxia research. Hypoxia 2015, 3, 35 - 43]. Eine Ursache hierfür liegt darin, dass es bis dato nicht möglich war, Sauerstoffkonzentrationen in der Mikroumgebung dreidimensionaler Aggregate und Gewebe genau messen und regeln zu können. Die aus der Literatur bekannten Angaben über die Gewebesauerstoffkonzentrationen sind darüber hinaus mit einer Unsicherheit behaftet, da sie häufig mit Sauerstoff-verbrauchenden Elektroden gemessen wurden, die die physiologischen Sauerstoffkonzentration in Geweben nicht exakt erfassen können.

Bisherige Systeme zur in vitro-Sauerstoffmessung ermöglichen in der Regel nur die Messung in 2D-Zellkulturen oder eine globale Messung von 3D-Zellkulturen (z.B. Agilent Seahorse XF). Außerdem ist mit solchen Systemen bisher keine parallele oder simultane Messung weiterer physiologisch relevanter Parameter möglich. Die Entwicklung biologischer Modellsysteme auf Basis multizellulärer Aggregate, beispielsweise Sphäroide bzw. Organoide, schreitet zwar immer weiter voran, jedoch sind entweder die Methoden zur Charakterisierung der Aggregate 2D-Kultur-basierten Protokollen entlehnt und deshalb nicht geeignet 3D-Kulturen zu analysieren oder es gibt bislang keine adäquaten Methoden zur Charakterisierung. Dies trifft vor allem auf die Charakterisierung der Herz-(Neben)Wirkung von Wirkstoffkandidaten oder für Anwendungen im Bereich von REACH (Registration, Evaluation, Authorisation of Chemicals) zu, insbesondere in Hinblick auf die ischämische Toxizität, für die aktuell noch keine geeigneten in vitro-Systeme verfügbar sind.

Im Bereich der Toxizitätsuntersuchungen gibt es zahlreiche in vitro-Systeme. Auch hier besteht das Problem, dass sich die Daten aus in vitro-Versuchen nur eingeschränkt auf die in vivo-Situation übertragen lassen. Dies liegt unter anderem daran, dass O$_2$ als wichtigster Kulturparameter nur selten kontrolliert wird. Gerade in dreidimensionalen Ansätzen ist eine zuverlässige Messung von Sauerstoff in der direkten Mikroumgebung der Gewebe erforderlich, da diese deutlich organotypischere Werte liefern als globale Sauerstoffmessungen und so zu einer deutlich besseren Übertragbarkeit auf die in vivo-Situation führen.

Ein weit verbreitetes Messsystem ist das Seahorse XF-Gerät von Agilent. Dieses kann unter anderem Sauerstoff messen und beispielsweise über den sogenannten Mito-Stress-Test Aussagen über den Einfluss von Chemikalien auf die Zellatmung treffen. Jedoch ist dieses System für zweidimensionale Zellkultur optimiert und misst auch nur global den Sauerstoffgehalt in einem Versuchsansatz. Eine gleichzeitige mikroskopische Betrachtung mehrerer physiologisch relevanter Parameter ist nicht möglich. Ein weiteres am Markt befindliches System (CYRIS®flox, Fa. INCYTON®) misst ebenfalls Sauerstoff und bietet die Möglichkeit zur Mikroskopie und Impedanzmessung, ist jedoch ebenfalls nur für 2D-Zellkulturen geeignet.

Ein Verfahren, um Sauerstoff in der Mikroumgebung von 3D-Zellkulturen oder Sphäroiden zu bestimmen, wird in der unveröffentlichten Europäischen Patentanmeldung EP23180517.7 beschrieben. Das hierin beschriebene Verfahren basiert auf den darin beschriebenen dreidimensionalen Strukturen auf Basis funktionalisierter Sensorfolien und hat die darin beschriebenen Methoden für die spezielle Anwendung an 3D-Zellkulturen von Kardiomyozyten und an Herzorganoiden weiterentwickelt, um neue und verbesserte in vitro-Modelle zur Bestimmung von Kardiotoxizität und ischämischer Toxizität bereitzustellen. Dabei können in den hierin beschriebenen in vitro-Modellen insbesondere auch mehrere physiologisch relevante Parameter mikroskopisch gemessen werden, um so in einem einfachen und effizienten Verfahren spezifisch die ischämische Toxizität zu bestimmen. Insbesondere die Messung von Sauerstoff in Kombination mit der Untersuchung von intrazellulären Calciumkonzentrationen bzw. von Calciumtransienten zur Gewinnung von Aussagen zum Calciumstoffwechsel bzw. Kontraktilität von Herzmuskelzellen bietet eine Erweiterung und Verbesserung der bisher beschriebenen Verfahren, insbesondere bei der Untersuchung der ischämischen Toxizität oder Kardiotoxizität.

Arzneimittel oder sonstige Wirkstoffe, sowie Chemikalien im Allgemeinen, können unterschiedlichste Einflüsse auf Organismen, also auch auf Zellaggregate haben. Daher ist eine Betrachtung einzelner Parameter für eine Beurteilung hinsichtlich Toxizität in der Regel nicht ausreichend. Das oben diskutierte Agilent Seahorse System kann zwar parallel Sauerstoff und den pH-Wert messen, ermöglicht aber keine mikroskopische Betrachtung und eignet sich nicht für Messungen an 3D-Aggregaten. Ein weiteres System, das CYRIS®flox System der Firma INCYTON® ermöglicht zwar generell die Mikroskopie und Sauerstoffmessung, ist aber ebenfalls nicht für 3D-Zellkulturen ausgelegt.

Das hierin beschriebene Verfahren ermöglicht eine parallele bzw. simultane mikroskopische Betrachtung verschiedener physiologisch relevanter Parameter an 3D-Zellaggregaten, insbesondere Kardiomyozytenaggregaten und Herzorganoiden, im Gegensatz sowohl zum Seahorse XF als auch zum CYRIS®flox System, die nur zweidimensional messen. In dem erfindungsgemäßen Verfahren können die verwendeten dreidimensionalen Strukturen auf Basis Fluorophor-dotierter Sauerstoffsensitiver Sensorfolien in Form von Mikrokavitäten mit hoher Dichte eingesetzt werden, wodurch eine Vielzahl von Zellaggregaten gleichzeitig betrachtet werden kann. Diese sind eindeutig über die fixe Position der Mikrokavitäten identifizierbar, sodass auch Langzeitexperimente über automatisierte Mikroskop-Plattformen möglich sind. Die hohe Anzahl an Parallelmesspunkten ermöglicht im Gegensatz zu anderen Systemen reproduzierbarere Aussagen, da jeder Datenpunkt mit einer Vielzahl an Wiederholungen simultan aufgenommen werden kann.

Ein weiterer Vorteil des hierin beschriebenen Verfahrens gegenüber den bestehenden Systemen der Fa. Agilent bzw. INCYTON® liegt darin, dass das hierin beschriebene in vitro-Verfahren unter physiologischen Bedingungen durchgeführt werden kann. Die hier beschriebenen bekannten Systeme setzen geschlossene Platten ein, was während der Versuchsdauer zu einer Hypoxie führen kann. Durch das offene Format der im erfindungsgemäßen Verfahren eingesetzten Assay-Plattform ist eine Begasung und so das Einstellen physiologischer Parameter möglich. Dies ermöglicht auch Assays über eine längere Versuchsdauer.

Das hierin beschriebene erfindungsgemäße Verfahren ermöglicht die Untersuchung von Stammzell-basierten Kardiomyozytenaggregaten sowie von Herzorganoiden, auch als "heart forming organoids" (HFOs) bezeichnet. Durch die Möglichkeit Sauerstoff in der Mikroumgebung der Gewebe zu bestimmen, kann beispielsweise der Einfluss von Wirkstoffen oder Chemikalien auf die mitochondriale Atmung untersucht werden. Da die in dem Verfahren eingesetzten funktionalisierten Sensorfolien eine gleichzeitige mikroskopische Auswertung ermöglichen, ist mit dem hierin beschriebenen Verfahren auch die Messung intrazellulärer Calciumkonzentrationen, z.B. über "Genetically Engineered Calcium Indicators" (GECI), möglich. Die gleichzeitige, d.h. parallele oder simultane, Betrachtung der Sauerstoffkonzentrationen sowie der Schlagfrequenz von Herzmuskelzell-Aggregaten über die intrazellulären Calciumkonzentrationen in derselben Zellkultur, ermöglichen die Generierung vollkommen neuer Datensätze und eine bessere Charakterisierung von Chemikalien bzw. Wirkstoffkandidaten hinsichtlich ihrer Kardiotoxizität.

Geeignete Herzorganoide (Heart Forming Organoids; HFOs), die in dem hierin beschriebenen *in* vitro-Verfahren eingesetzt werden können, sowie deren Herstellung beschreibt das Europäische Patent EP3765599B1.

## AUFGABENSTELLUNG

Die Aufgabe der vorliegenden Erfindung bestand darin, ein neues *in* vitro-Verfahren zur Bestimmung physiologisch bedeutender Analyte und/oder Analytgradienten, wie insbesondere von Sauerstoff, in 3D-Zellkulturen zu entwickeln, womit verbesserte *in* vitro-Modelle zur Untersuchung der ischämischen Toxizität und/oder zur Bestimmung von Kardiotoxizität bereitgestellt werden können. In einem weiteren Aspekt der Erfindung sollte das neue Verfahren die Messung von Sauerstoff oder Sauerstoffgradienten in Kombination mit einem oder mehreren weiteren für die Untersuchung von ischämischer Toxizität und Kardiotoxizität relevanten Analyten in derselben Zellkultur, ermöglichen, wie insbesondere eine parallele oder simultane Messung von Sauerstoff und intrazellulären Calciumkonzentrationen zur Bestimmung von Calciumtransienten (zur Untersuchung des Calciumstoffwechsels), sowie ggf. eine parallele oder simultane Messung von $CO_2$-, Glukosekonzentration und pH-Werten.

Das neue Verfahren sollte weiterhin geeignet sein, alle Schritte, von der 3D-Zellkultivierung bis hin zu Langzeitmessungen, idealerweise in automatisierten Verfahren mit hohem Probendurchsatz, einfach und effizient ausführen zu können. Weiterhin sollte ein Verfahren bereitgestellt werden, mit dem die zu bestimmenden Parameter unter möglichst physiologischen Bedingungen erfasst werden können.

Diese Aufgaben wurden durch das hierin beschriebene neue Verfahren gelöst, welches die *in* vitro-Messung von Sauerstoffkonzentrationen und/oder Sauerstoffkonzentrationsgradienten in 3D-Zellkulturen von Kardiomyozytenaggregaten oder Herzorganoiden in dreidimensionalen Strukturen aus Fluorophor-dotierten Sauerstoff-sensitiven Sensorfolien ermöglicht.

Das erfindungsgemäße Verfahren ermöglicht außerdem auch die Kultivierung und Analytbestimmung in den in dem erfindungsgemäßen Verfahren eingesetzten dreidimensionalen Strukturen aus funktionalisierten Sensorfolien. Eine Überführung der kultivierten Zellen in die Messumgebung wird damit entbehrlich.

Durch Ausbildung der dreidimensionalen Strukturen in Form von Mikrokavitäten können auf der Fläche einer Messeinheit, mehrere Hundert bis Tausend Mikrokavitäten generiert und somit auch mehrere Hundert bis Tausend 3D-Zellkulturen kultiviert und untersucht werden. Für jede einzelne dieser 3D-Zellkulturen können Informationen über die Analytkonzentrationen und -gradienten gewonnen und in Vergleichsversuchen oder Langzeituntersuchungen gemessen werden. Messungen in dieser Größenordnung sind mit den im Stand der Technik beschriebenen Ansätzen in der Praxis nicht umsetzbar. Außerdem ist mit dem erfindungsgemäßen Verfahren erstmals das Erfassen von mehreren unterschiedlichen Analyten und/oder Analytgradienten mit demselben Messaufbau innerhalb derselben Zellkultur möglich, sodass relevante Analyte parallel bzw. simultan, und damit wesentlich effizienter, untersucht werden können.

Mit dem erfindungsgemäßen Verfahren und dessen Eignung zur Untersuchung von 3D-Zellkulturen von Kardiomyozytenaggregaten oder Herzorganoiden kann somit ein verbessertes *in* vitro-Modell für die Bestimmung der ischämischen Toxizität oder Kardiotoxizität bereitgestellt werden, welches eine bessere Übertragbarkeit der *in vitro*-Ergebnisse auf die *in* vivo-Situation erlaubt.

## BESCHREIBUNG DER ERFINDUNG

Die vorliegende Erfindung wird nachfolgend näher beschrieben und umfasst insbesondere die folgenden Aspekte:

[1] Verfahren zur *in* vitro-Messung von Sauerstoffkonzentrationen und/oder Sauerstoffkonzentrationsgradienten in 3D-Zellkulturen von Kardiomyozyten oder an Herzorganoiden (HFOs), umfassend

a) Bereitstellung von dreidimensionalen Strukturen, die aus Fluorophor-dotierten Sauerstoffsensitiven Sensorfolien geformt sind,
b) Kultivierung der 3D-Zellkulturen in Form von Kardiomyozytenaggregaten oder von Herzorganoiden in den dreidimensionalen Fluorophor-dotierten Sauerstoff-sensitiven Sensorfolien oder Überführung der bereits kultivierten 3D-Zellkulturen oder der Herzorganoide in die dreidimensionalen Fluorophor-dotierten Sauerstoff-sensitiven Sensorfolien,
c) Sauerstoffmessung in der 3D-Zellkultur mittels optischer Methoden, wie der Mikroskopie,
d) Erfassung und Auswertung der Messdaten.

[2] Verfahren gemäß [1], worin zusätzlich zur Messung der Sauerstoffkonzentration und/oder von Sauerstoffkonzentrationsgradienten die Messung von intrazellulären Calciumkonzentrationen und/oder -Transienten in derselben Zellkultur erfolgt.

[3] Verfahren gemäß [2], worin die Messung von intrazellulären Calciumkonzentrationen und/oder-Transienten über Genetically Engineered Calcium Indicators (GECI) erfolgt.

[4] Verfahren gemäß einem von [1] bis [3], worin zusätzlich einer oder mehrere der Parameter $CO_2$, Glukose und pH-Wert in der 3D-Zellkultur gemessen werden.

[5] Verfahren gemäß einem von [1] bis [4], worin es sich bei den dreidimensionalen Strukturen, die aus Fluorophor-dotierten Sauerstoff-sensitiven Sensorfolien geformt sind, um Mikrokavitäten oder Mikrostrukturen handelt, die zur Aufnahme und/oder Kultivierung von Zellen geeignet sind.

[6] Verfahren gemäß einem von [1] bis [5], worin die dreidimensionalen Strukturen eine zusätzliche Funktionalisierung mit Calcium, $CO_2$-, Glukose- und/oder pH-Sensitivität aufweisen.

[7] Verfahren gemäß einem von [1] bis [6], worin die Fluorophor-dotierten Sauerstoff-sensitiven Sensorfolien mittels einer Plasmabehandlung und/oder durch Beschichtung mit Beschichtungsmitteln, wie extrazellulärer Matrix (Collagen), modifiziert sind.

[8] Verfahren gemäß einem von [1] bis [7], worin die 3D-Zellkulturen von Kardiomyozyten oder die Herzorganoide (HFOs) aus pluripotenten, omnipotenten oder multipotenten Stammzellen, bevorzugt aus humanen pluripotenten, omnipotenten oder multipotenten Stammzellen, bevorzugter aus humanen pluripotenten Stammzellen differenziert oder aus primären Kardiomyozyten generiert werden.

[9] Verfahren gemäß einem von [1] bis [8], worin die *in* vitro-Messung der Analyten an Herzorganoiden (HFOs) erfolgt, welche dadurch gekennzeichnet sind, dass diese eine erste, einen inneren Teil bildende und Hohlräume aufweisende Schicht aufweisen, die zumindest teilweise von einer zweiten, Endothelzellen und Kardiomyozyten umfassenden Schicht umgeben ist, die zumindest teilweise von einer dritten, Kardiomyozyten und Epikardzellen umfassenden Schicht umgeben ist, die zumindest teilweise von einer vierten, Fibroblastenzellen umfassenden Schicht umgeben ist.

[10] Verfahren gemäß [9], worin die Herzorganoide dadurch gekennzeichnet sind, dass die Hohlräume des inneren Teils Vormagenendoderm, Blutgefäße und hämogenes Endothel enthalten.

[11] Verfahren gemäß einem von [1] bis [10], worin die Kultivierung der 3D-Zellkultur oder der Herzorganoide und die Messung der Analyten in den dreidimensionalen Strukturen erfolgt.

[12] Verfahren gemäß einem von [1] bis [11], worin die mikroskopische Analytmessung mittels Konfokalmikroskopie, insbesondere Fluoreszenzlebenszeit-Mikroskopie erfolgt.

[13] Verfahren gemäß einem von [1] bis [12], worin die Analytmessung an verschiedenen Messpunkten (ortsaufgelöst) unter Erfassung des Analytgradienten erfolgt.

[14] Verfahren gemäß einem von [1] bis [13], worin die dreidimensionalen Strukturen, die aus Fluorophor-dotierten Sauerstoff-sensitiven Sensorfolien geformt sind, in Form von Mikrokavitätenarrays, Mikrokanälen oder anderen Mikrovertiefungen für die Anwendung (den Einsatz) in Mikrotiterplatten, in Zellkulturplatten oder in Zellkulturinserts mit einem oder mehreren Kompartimenten vorliegen.

[15] Verfahren gemäß einem von [1] bis [14], worin die dreidimensionalen Strukturen, die aus Fluorophor-dotierten Sauerstoff-sensitiven Sensorfolien geformt sind, in Form von Mikrokavitäten vorliegen, worin die Kavitäten eine Fase an der Öffnung der Kavitäten aufweisen, worin die Fasen bevorzugt einen Öffnungswinkel von 1 bis 179°, bevorzugter von 20 bis 120°, noch bevorzugter von 30 bis 60° aufweisen.

[16] Verfahren gemäß einem von [1] bis [15], worin die Sauerstoff-sensitiven Sensorfolien Fluorophor-dotierte Polymerträgerfolien oder Polycarbonatträgerfolien sind, worin die Fluorophor-Dotierung in Form einer auf die Trägerfolien aufgebrachten Fluorophor-Beschichtung oder in Form von in Hydrogelen oder in Polymermatrizes immobilisierten Fluorophoren vorliegt.

[17] Verfahren gemäß einem von [1] bis [16], worin die Fluorophore ausgewählt sind aus der Gruppe der Metallkomplexe, umfassend insbesondere Platin-II-, Palladium-II- und Ruthenium-II-Komplexe.

[18] Verfahren gemäß einem von [1] bis [17], worin die dreidimensionalen Strukturen Bereiche mit zusätzlicher Funktionalisierung mit $CO_2$-, Glucose- und/oder pH-Sensitivität aufweisen, welche in den dreidimensionalen Strukturen räumlich getrennt von den Sauerstoff-sensitiven funktionalisierten Bereichen angeordnet sind.

[19] Verfahren gemäß [18], worin die zusätzlichen funktionalisierten Bereiche mit $CO_2$-, Glucose- und/oder pH-Sensitivität in Form von Spots ausgebildet sind.

[20] Verfahren gemäß einem von [1] bis [19], worin die Erfassung und Auswertung der Messdaten mit einem oder mehreren Elementen zur automatisierten Erfassung von Messdaten, Datenverarbeitung, Datenauswertung und Datenausgabe erfolgt.

[21] Verfahren gemäß [20], wobei die Datenverarbeitung und Datenauswertung eine KIgestützte Evaluation der Messdaten umfasst.

[22] Verfahren gemäß [21], worin zur Auswertung der Messdaten eine KI einen Datenabgleich der erhobenen Messdaten mit bereits vorhandenen Daten ausführt.

[23] Verwendung des Verfahrens gemäß einem von [1] bis [22] zur Untersuchung des Einflusses von Testsubstanzen auf die mitochondriale Atmung, zur Bestimmung der ischämischen Toxizität und/oder zur Bestimmung der Kardiotoxizität.

[24] Verwendung nach [23] zur Klassifikation von Wirkstoffen und/oder Wirkstoffkandidaten in der pharmazeutischen oder kosmetischen Industrie oder zur Evaluation von Chemikalien nach dem REACH-(Registration, Evaluation, Authorisation of Chemicals) System.

## DETAILLIERTE BESCHREIBUNG DER ERFINDUNG

Die vorliegende Erfindung betrifft ein Verfahren zur *in vitro*-Messung von Sauerstoffkonzentrationen und/oder Sauerstoffkonzentrationsgradienten in 3D-Zellkulturen von Kardiomyozyten oder an Herzorganoiden (HFOs), umfassend

a) Bereitstellung von dreidimensionalen Strukturen, die aus Fluorophor-dotierten Sauerstoff-sensitiven Sensorfolien geformt sind,
b) Kultivierung der 3D-Zellkulturen in Form von Kardiomyozytenaggregaten oder von Herzorganoiden in den dreidimensionalen Fluorophor-dotierten Sauerstoff-sensitiven Sensorfolien oder Überführung der bereits kultivierten 3D-Zellkulturen oder der Herzorganoide in die dreidimensionalen Fluorophor-dotierten Sauerstoff-sensitiven Sensorfolien
c) Sauerstoffmessung in der 3D-Zellkultur mittels optischer Methoden, wie der Mikroskopie,
d) Erfassung und Auswertung der Messdaten.

Die Erfindung umfasst außerdem einen Assay, womit das erfindungsgemäße Verfahren durchgeführt werden kann.

Ein wesentlicher Aspekt des erfindungsgemäßen Verfahrens liegt in der Möglichkeit der Untersuchung dreidimensionaler Zellkulturen, so dass das Verfahren geeignet ist, Untersuchungen an Kardiomyozytenaggregaten oder Herzorganoiden durchzuführen. Der Begriff "Organoide" bezeichnet dabei eine Zellaggregatstruktur, die artifiziell erzeugt (kultiviert) worden ist. Organoide besitzen organähnliche Eigenschaften in Abhängigkeit davon, unter welchen Bedingungen sie kultiviert worden sind.

Die in dem erfindungsgemäßen Verfahren eingesetzten 3D-Zellkulturen von Kardiomyozyten bzw. die Herzorganoide (HFOs) können prinzipiell aus pluripotenten, omnipotenten oder multipotenten Stammzellen, bevorzugt aus humanen pluripotenten, omnipotenten oder multipotenten Stammzellen, bevorzugter aus humanen, induzierten pluripotenten Stammzellen differenziert oder aus primären Kardiomyozyten generiert werden..

In dem erfindungsgemäßen Verfahren können beispielsweise Herzorganoide eingesetzt werden, wie sie in dem Europäischen Patent EP3765599B1 beschrieben und charakterisiert werden. Die Herstellung bzw. Kultivierung solcher HFOs in den dreidimensionalen Strukturen aus Sauerstoff-sensitiven Sensorfolien gemäß dem erfindungsgemäßen Verfahren kann nach dem in der EP3765599B1 beschriebenen Verfahren erfolgen, welche diesbezüglich vollumfänglich von der vorliegenden Erfindung umfasst wird. Die HFOs gemäß der EP3765599B1 sind beispielsweise dadurch gekennzeichnet, dass diese eine erste, einen inneren Teil bildende und Hohlräume aufweisende Schicht aufweisen, die zumindest teilweise von einer zweiten, Endothelzellen und Kardiomyozyten umfassenden Schicht umgeben ist, die zumindest teilweise von einer dritten, Kardiomyozyten und Epikardzellen umfassenden Schicht umgeben ist, die zumindest teilweise von einer vierten, Fibroblastenzellen umfassenden Schicht umgeben ist. Die darin beschriebenen Herzorganoide sind außerdem dadurch gekennzeichnet, dass die Hohlräume des inneren Teils Vormagenendoderm, Blutgefäße und hämogenes Endothel enthalten.

Das erfindungsgemäße Verfahren und der erfindungsgemäße Assay basieren somit auf der Nutzung dreidimensionaler Strukturen, die aus Fluorophor-dotierten Sauerstoff-sensitiven Sensorfolien geformt sind und die vorliegende Erfindung stellt eine Plattform zur Charakterisierung schlagender Kardiomyozytenaggregate bzw. sog. heart forming organoids (HFOs) bereit. Derartige HFOs beschreiben außerdem Drakhlis et al. und Halloin et al. [Drakhlis, L., S. Biswanath, C. M. Farr, V. Lupanow, J. Teske, K. Ritzenhoff, A. Franke, F. Manstein, E. Bolesani, H. Kempf, S. Liebscher, K. Schenke-Layland, J. Hegermann, L. Nolte, H. Meyer, J. de la Roche, S. Thiemann, C. Wahl-Schott, U. Martin, and R. Zweigerdt. 2021. 'Human heart-forming organoids recapitulate early heart and foregut development', Nat Biotechnol.; Drakhlis, Lika, Santoshi Biswanath Devadas, and Robert Zweigerdt. 2021. 'Generation of heart-forming organoids from human pluripotent stem cells', Nature Protocols, 16: 5652-72; Halloin, Caroline, Kristin Schwanke, Wiebke Löbel, Annika Franke, Monika Szepes, Santoshi Biswanath, Stephanie Wunderlich, Sylvia Merkert, Natalie Weber, Felix Osten, Jeanne De La Roche, Felix Polten, Kai Christoph Wollert, Theresia Kraft, Martin Fischer, Ulrich Martin, Ina Gruh, Henning Kempf, and Robert Zweigerdt. 2019. 'Continuous WNT Control Enables Advanced hPSC Cardiac Processing and Prognostic Surface Marker Identification in Chemically Defined Suspension Culture', Stem Cell Reports, 13: 366-79.].

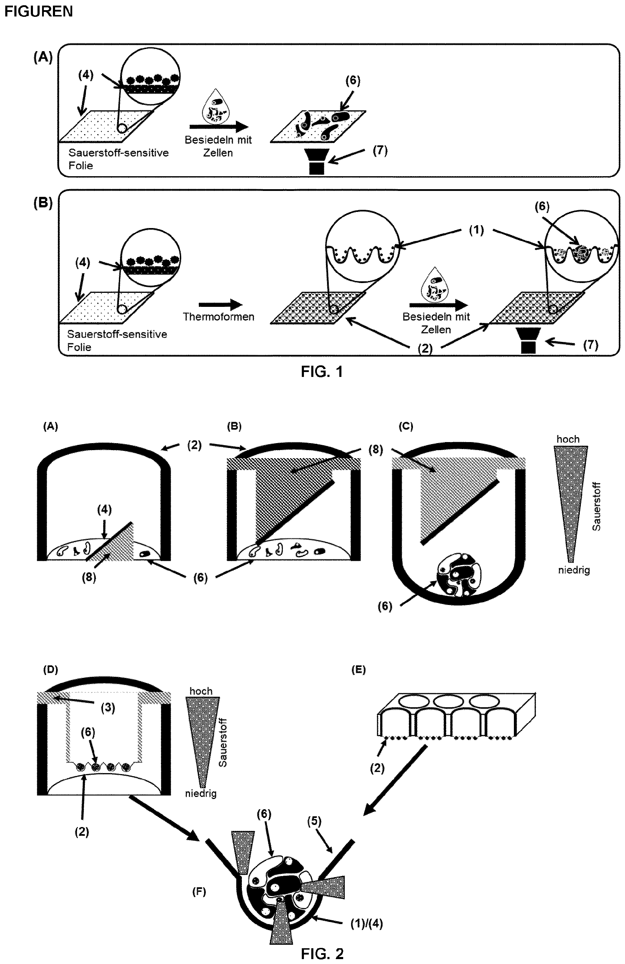

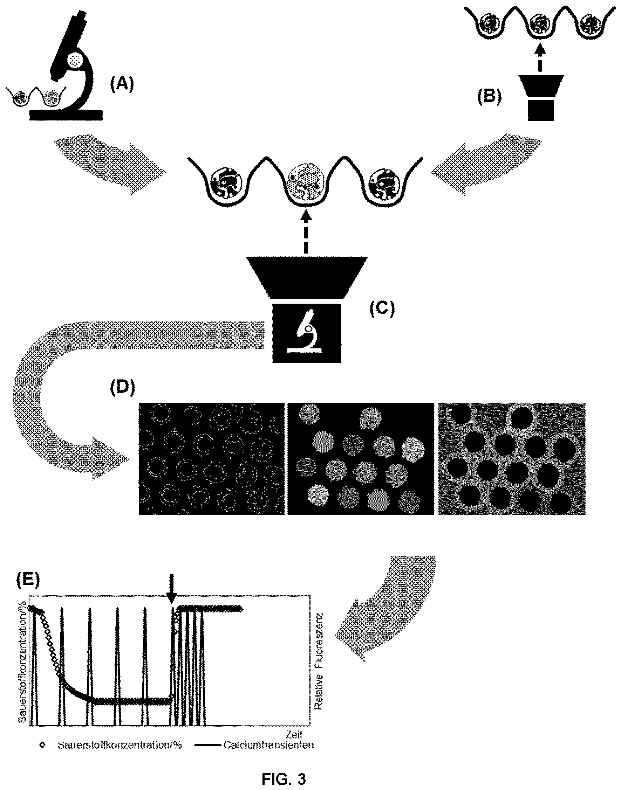

Das erfindungsgemäße Mess- und Kultivierungssystem basiert auf Sauerstoff-sensitiven Mikrokavitäten, in denen oben genannte Zellaggregate generiert und kultiviert werden können. Dadurch lassen sich die Sauerstoffkonzentrationen in unmittelbarer Mikroumgebung der Zellen bzw. Gewebe messen. Der Aufbau der erfindungsgemäß eingesetzten Assays mit den funktionalisierten dreidimensionalen Strukturen ermöglicht es, die gemessenen Analyten und/oder Analytgradienten in der dreidimensionalen Mikroumgebung um die 3D-Zellaggregate bzw. Organoide herum zu untersuchen. Dies ist insbesondere von Vorteil, wenn Analytgradienten bestimmt werden sollen. Weiterhin ermöglicht das erfindungsgemäß eingesetzte Messsystem es auch, globale Messungen über die gesamte dreidimensionale Struktur auszuführen. Das zugrundeliegende Messprinzip an 3D-Zellkulturen im Vergleich zu herkömmlichen 2D-Messverfahren illustrieren die Figuren 1 und 2. Figur 3 skizziert das erfindungsgemäße Verfahren zur Bestimmung ischämischer Toxizität.

Dazu werden die zu untersuchenden 3D-Zellkulturen wie Kardiomyozytenaggregate oder HFOs entweder direkt in den Mikrokavitätenarrays, z.B. in einem Zellkulturinsert, kultiviert oder sie werden außerhalb kultiviert und für die Messung in die Mikrokavitäten überführt. Ein besonderer Vorteil der erfindungsgemäß eingesetzten funktionalisierten Mikrokavitäten liegt in der Möglichkeit der Zellkultivierung und Messung innerhalb der Assay-Kavitäten. Die zur Bildung der Mikrokavitäten verwendeten funktionalisierten Sauerstoff-sensitiven Sensorfolien können durch geeignete Modifizierungen, wie z.B. Plasmabehandlung und/oder Beschichtung mit Beschichtungsmitteln, wie z.B. mit anti-adhäsiven Beschichtungslösungen oder mit extrazellulärer Matrix (z.B. Collagen), für die Kultivierung der 3D-Zellkulturen optimiert werden. Beispielsweise kann eine Beschichtung mit BIOFLOAT™ (faCellitate GmbH) erfolgen, wodurch die Bildung von Sphäroiden direkt in den Mikrokavitäten unterstützt wird.

Dabei umfasst das erfindungsgemäße Verfahren die Möglichkeit, in den Mikrokavitäten zunächst in einem vorgelagerten Kultivierungsschritt die Zellkultur zu kultivieren und anschließend die Analytmessung in derselben Kavität durchzuführen. Es ist auch möglich, dass die Analytbestimmung bereits während der Kultivierung erfolgt. Sofern hierin auf eine gleichzeitige oder simultane Kultivierung und Analytbestimmung in den Mikrokavitäten Bezug genommen wird, so sind damit auch Abläufe umfasst, worin zuerst die Zellkultivierung und Bildung der 3D-Zellkulturen erfolgt und anschließend die Analytbestimmung durchgeführt wird. Ebenso umfasst sind Abläufe, was in vielen Fällen vorteilhaft ist, worin bereits während der Kultivierungsphase eine Analytbestimmung durchgeführt und der Zellkultivierungsprozess über den Zeitverlauf untersucht wird. Dies ist durch die non-invasive Messmethode mittels Mikroskopie problemlos möglich. So lässt sich bereits während der Kultivierungsphase die Sauerstoffkonzentration bzw. der Sauerstoffgradient in der Kultur bestimmen und verfolgen. Damit sind auch Untersuchungen zum Langzeit-Einfluss von Testsubstanzen auf die Zellen möglich.

Langzeituntersuchungen werden außerdem dadurch ermöglicht, dass die in dem erfindungsgemäßen Verfahren eingesetzten Mikrokavitätenstrukturen in einem offenen Format zur Verfügung gestellt werden können. Durch dieses offene Format ist eine Begasung und so das Einstellen physiologischer Parameter möglich, was die Lebenszeit der zu untersuchenden Zellaggregate oder Organoide entschieden erhöht. So werden Untersuchungen realisierbar, die mehrere Wochen andauern können. Diese Eigenschaft des in dem erfindungsgemäßen Verfahren eingesetzten Assays mit den funktionalisierten dreidimensionalen Kultivierungs- und Mess-Strukturen bietet entscheidende Vorteile gegenüber den aus dem Stand der Technik bekannten Systemen, wie z. B. Systemen der Firma Agilent bzw. INCYTON®.

Da die eingesetzten dreidimensionalen funktionalisierten Strukturen in Form von Mikrokavitäten mit hoher Mikrokavitätendichte vorliegen können, sind mit dem erfindungsgemäßen Verfahren auch Hochdurchsatzexperimente durchführbar und es können zahlreiche gleiche oder unterschiedliche Zellaggregate oder Organoide simultan oder parallel untersucht werden. Da die dreidimensionalen Strukturen aus funktionalisierten Sensorfolien außerdem in räumlich getrennte Bereiche gegliedert angeordnet sein können, beispielsweise über die Anordnung und Aufbringung auf sogenannten Zellkulturinserts mit mehreren Kammern, lassen sich im Hochdurchsatz verschiedene Kulturen oder mehrere gleiche Kulturen und Kontrollkulturen jeweils gleichzeitig untersuchen. Durch die Möglichkeit der Langzeit- und Hochdurchsatzuntersuchung der 3D-Zellaggregate bzw. Organoide kann das erfindungsgemäße Verfahren eine hohe Informationsdichte bereitstellen. Durch die Aufnahme der Datenpunkte mit z.B. 20 bis 50 Wiederholungen liefert das System eine hohe Anzahl an Parallelmesspunkten, was im Gegensatz zu anderen Systemen reproduzierbarere Aussagen ermöglicht. Durch die Verwendung von dreidimensionalen Strukturen in Form von Mikrokavitäten können alle erhobenen Messwerte eindeutig den entsprechenden Zellaggregaten bzw. Organoiden zugeordnet werden, da sie eindeutig über die fixe Position der Mikrokavitäten identifizierbar sind. Ein weiterer Vorteil des eingesetzten Assays liegt darin, dass die Analyt-Messungen in dem erfindungsgemäßen Verfahren ortsaufgelöst erfolgen können. Dadurch dass das erfindungsgemäße Verfahren auf einer mikroskopischen Mess- und Bestimmungsmethode basiert, wie insbesondere auf der Nutzung der Konfokal- und/oder Fluoreszenzmikroskopie (Fluoreszenzlebenszeitmikroskopie), ist es insbesondere möglich, dreidimensional aufgelöste Konzentrationsgradienten, beispielsweise der Sauerstoffkonzentration, in der Mikroumgebung der untersuchten Zellaggregate bzw. Organoide zu untersuchen. Um die Untersuchung mittels (Fluoreszenz-)Mikroskopie zu ermöglichen, sollten die die dreidimensionalen Strukturen bildenden Sensorfolien ausreichend transparent sein. Eine ausreichende Transparenz sollte auch im Fall einer zusätzlichen Beschichtung (z.B. aufgrund Funktionalisierung oder für die Verbesserung der Zellkultivierung) erhalten bleiben.

Ein weiterer entscheidender Vorteil des in dem erfindungsgemäß eingesetzten Assays liegt in der Möglichkeit der simultanen oder parallelen Messung weiterer physiologisch relevanter Parameter in derselben Zellkultur. Dabei kann die Messung mehrerer Analyten in derselben Zellkultur bzw. in ein und demselben Kultivierungsansatz erfolgen. Eine solche gleichzeitige bzw. parallele oder simultane Messung umfasst die Messung unterschiedlicher Analyte im selben Kultur- oder Messansatz im zeitlichen Wechsel oder nacheinander oder simultan, also gleichzeitig, abhängig vom instrumentellen Aufbau und der instrumentellen Ausstattung des Assays.

Somit haben die Erfinder der vorliegenden Erfindung ein neues Untersuchungsverfahren entwickelt, worin an 3D-Zellkulturen von Kardiomyozytenaggregaten bzw. Herzorganoiden zusätzlich zur Messung der Sauerstoffkonzentration und/oder von Sauerstoffkonzentrationsgradienten die Messung von intrazellulären Calciumkonzentrationen in derselben Zellkultur vorgenommen werden kann. Die Bestimmung intrazellulärer Calciumkonzentrationen erfolgt dabei ebenfalls fluoreszenzmikroskopisch und kann somit gleichzeitig bzw. parallel zur Sauerstoffmessung im gleichen Messaufbau durchgeführt werden (siehe auch Figur 3). Dabei bietet das hierin beschriebene neue Verfahren die Möglichkeit, z.B. über von extern zugesetzte Calciumsensormoleküle oder sogenannte "genetically engineered calcium indicators" (GECI), in den eingesetzten Zellen Calciumtransienten zu messen, mit deren Hilfe die intrazellulären Calciumkonzentrationen, Calciumtransienten (der Calciumstoffwechsel) und darüber die Schlagfrequenz bestimmt werden können. Bei solchen GECIs handelt es sich um Proteine deren DNA-Informationen zuvor über Transfektion in die untersuchten Zellen eingeschleust wurden. Die daraufhin exprimierten GECI-Proteine reagieren bei Calciumanwesenheit mit einer Änderung ihrer spektroskopischen Eigenschaften und bewirken so z.B. eine Änderung in der Fluoreszenz, beispielsweise eine Fluoreszenzemission bei Calciumbindung. Solche Änderungen der fluoreszenzspektroskopischen Eigenschaften lassen sich mit dem erfindungsgemäßen Verfahren messen und damit intrazelluläre Calciumkonzentrationen, Calciumtransienten (der Calciumstoffwechsel) und darüber die Schlagfrequenz der untersuchten Zellen untersuchen. Das Grundprinzip und darauf basierende Messverfahren sind in der Literatur beschrieben [Ricci Signorini, M. E., M. Szepes, A. Melchert, M. Bakar, S. Merkert, A. Haase, G. Gohring, U. Martin, and I. Gruh. 2022. 'Generation of human induced pluripotent stem cell lines encoding for genetically encoded calcium indicators RCaMP1h and GCaMP6f, Stem Cell Res, 60: 102697].

In einem besonders bevorzugten Aspekt der Erfindung werden somit GECI-modifizierte Kardiomyocyten oder HFOs eingesetzt, also solche in welche zuvor über Transfektion die DNA-Information von GECI-Proteinen eingeschleust wurde, so dass die daraufhin exprimierten GECI-Proteine in den eingesetzten Zellen hinsichtlich ihrer Reaktion auf Calcium detektierbar werden.

Neben GECIs lassen sich auch andere Indikatoren in die Zellen einschleusen, die Aufschluss z.B. über physiologische, biochemische oder Expressionsveränderungen im Genom geben. Die bisher einzigartige Kombination der Messung der Sauerstoffkonzentrationen in 3D und die Messung der Calciumtransienten ermöglichen Aussagen über Substanz-induzierte Änderungen im Verhalten der Kardiomyozyten. So ist es möglich Chemikalien bzw. Wirkstoffkandidaten hinsichtlich ihrer Kardiotoxizität zu charakterisieren und genauere Untersuchungen zur ischämischen Toxizität durchzuführen. Über eine solche zusätzlich zur Sauerstoffmessung durchgeführte Untersuchung der Calciumtransienten kann z.B. ein Substanz-induzierter Einfluss auf die Schlagfrequenz der eingesetzten Kardiomyozytenaggregate bzw. Herzorganoide untersucht werden. Diese Untersuchung erfolgt in dem eingesetzten Verfahren fluoreszenzmikroskopisch, wobei das verwendete Fluoreszenzmikroskop in das System zur Sauerstoffmessung integriert werden kann, wodurch der Vorteil der simultanen Messung ermöglicht wird.

Neben der Kombination der Sauerstoffmessung mit der Bestimmung intrazellulärer Calciumkonzentrationen bzw. Calciumtransienten können alternativ oder zusätzlich auch andere bzw. weitere physiologisch relevante Parameter in Kombination mit der Sauerstoffbestimmung in den Zellkulturen bestimmt werden. Beispiele weiterer Analyte umfassen insbesondere $CO_2$, Glukose und der pH-Wert, welche in beliebigen Kombinationen miteinander zusätzlich zur Sauerstoffmessung in der Zellkultur untersucht werden können.

Grundsätzlich kann eine solche zusätzliche Messung weiterer Analyten, wie Calcium-, $CO_2$-, Glukose-Konzentrationen und/oder pH-Wert, in Kombination mit der Sauerstoffmessung, parallel oder simultan (gleichzeitig) erfolgen oder im zeitlichen Wechsel bzw. nacheinander innerhalb desselben Kultur- oder Messansatzes.

Die kombinierte (gleichzeitige, parallele bzw. simultane) Untersuchung verschiedener Analyte kann beispielsweise dadurch erzielt werden, dass die eingesetzten dreidimensionalen Strukturen, die z.B. in Form funktionalisierter Sauerstoff-sensitiver Mikrokavitäten vorliegen, eine oder mehrere gleiche oder unterschiedliche zusätzliche Funktionalisierungen zur Bestimmung von $CO_2$, Glukose und/oder für pH-Messungen aufweisen (sogenannte Funktionalisierungs- oder Sensorspots). Solche zusätzlichen Funktionalisierungs-Spots können ebenfalls aus Fluorophor-dotierten Sensorfolien gebildet werden. Es ist dabei möglich, dass die die Mikrokavitäten bildenden funktionalisierten Sauerstoff-sensitiven Sensorfolien und die zusätzlichen Sensorspots eine gleiche oder eine unterschiedliche Fluorophor-Dotierung aufweisen. In einer bevorzugten Ausführungsform ist diese zusätzliche Fluorophor-Dotierung der Sensorspots sensitiv gegenüber einem anderen Analyten als die Sauerstoff-sensitive dreidimensionale Struktur der Mikrokavitäten, um so die kombinierte und parallele oder simultane Messung von mindestens einem zusätzlichen Analyten zu ermöglichen. Je nach Wahl der Anordnung dieser zusätzlichen Sensorspots zu den Sauerstoff-sensitiven Mikrokavitäten ist die Analytbestimmung an unterschiedlichen Messpunkten im System möglich.

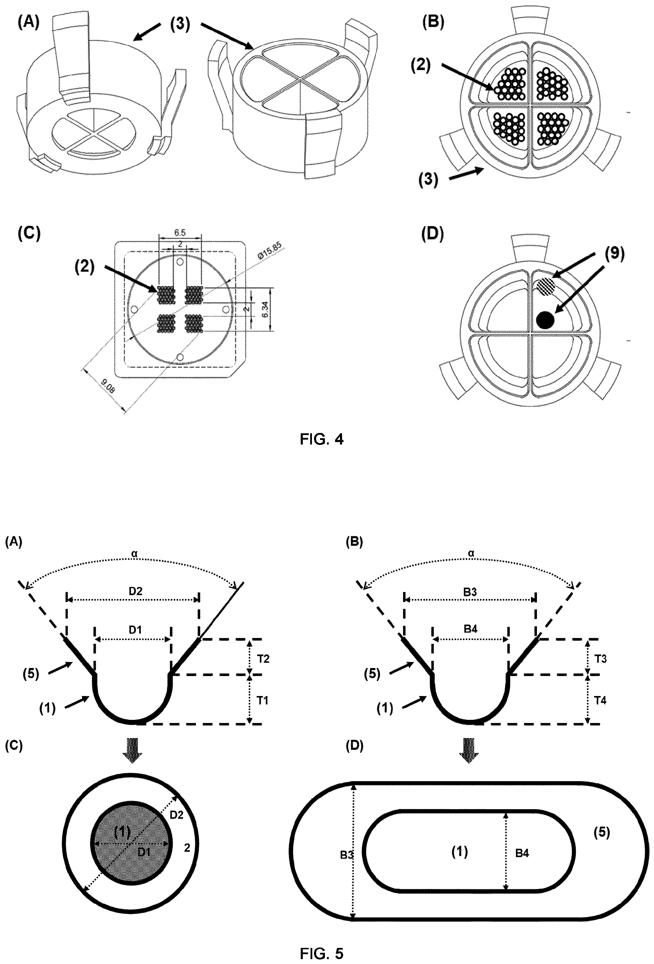

In einer geeigneten und bevorzugten Ausführungsform des erfindungsgemäßen Verfahrens werden die aus der funktionalisierten Sauerstoff-sensitiven Sensorfolie gebildeten Mikrokavitäten in ein Zellkulturinsert integriert. Auf solchen Zellkulturinserts können dann auch die zusätzlichen (gleichen oder unterschiedlichen) Sensorspots für die Messung weiterer Analyte, z.B. räumlich voneinander getrennt, aufgebracht werden. Eine solche Ausgestaltung zeigt Figur 4(D).

Beispielsweise ist es möglich, Zellkulturinserts zu verwenden, welche eine Unterteilung in mehrere Kompartimente aufweisen. Solche in Kompartimente aufgeteilten Zellkulturinserts ermöglichen verschiedene Konfigurationen der Assays für eine hohe Variabilität des erfindungsgemäßen Verfahrens. Eine beispielhafte Anordnung mit 4 Kompartimenten zeigt die Figur 4. Beispielsweise kann so, je nach Anzahl der Kompartimente, eine parallele Messung von zwei und mehr Ansätzen mit einer Testsubstanz und zusätzlich einer Positiv- und einer Negativkontrolle erfolgen. In einer anderen Variante ist beispielsweise die Messung der Testsubstanz in verschiedenen Konzentrationen möglich. In einer weiteren Variante können auch unterschiedliche Testsubstanzen parallel untersucht werden. Je nach Anzahl der Kompartimente können die Untersuchungsansätze variabel an die Testerfordernisse angepasst werden.

Besonders geeignet für parallele oder simultane Messungen mehrerer Analyten sind solche Anordnungen, worin die dreidimensionalen Strukturen aus Sauerstoff-sensitiven Sensorfolien und die zusätzlichen Bereiche mit Funktionalisierung mit $CO_2$-, Glukose- und/oder pH-Sensitivität räumlich getrennt voneinander angeordnet sind.

Solche weiteren Sensorspots können genutzt werden, um z.B. $CO_2$ oder den pH-Wert global für den gesamten Testansatz, z.B. das gesamte Test-Insert, zu bestimmen. Über den pH-Wert lässt sich auch die sogenannte extracellular acidification rate (ECAR) bestimmen. Dabei kann zum einen der pH-Wert direkt gemessen werden, wenn eine pH-sensitive Folie verwendet wird. Bei solchen Sensorspots handelt es sich um Bereiche, die mit einer entsprechend dem zu bestimmenden Analyten sensitiven Funktionalisierung ausgestattet sind, beispielsweise indem sie eine $CO_2$- bzw. pH-sensitive Fluorophor-Dotierung aufweisen, z.B. in Form einer Beschichtung. Diese Spots können unmittelbar neben den Sauerstoff-sensitiven Mikrokavitäten oder im Bereich der Zellkulturinserts, die die Mikrokavitäten aufnehmen, angeordnet werden. Es ist aber auch möglich, dass solche zusätzlichen Funktionalisierungsspots auf den funktionalisierten Sensorfolien in Form von Mikroarrays aufgebracht sind, beispielsweise indem sie mit einem weiteren Fluorophor beschichtet sind, beispielsweise neben oder innerhalb der Mikrokavitäten. Diese Funktionalisierungsspots sind gegenüber einem anderen Analyten sensitiv als die die Mikrokavitäten bildenden Sauerstoff-sensitiven Sensorfolien. Zum Beispiel kann über die zusätzliche Messung von pH-Wert und $CO_2$-Konzentration zwischen den Kontributionen der Glykolyse- oder zellatmungsabhängigen ECAR unterschieden werden.

Wie bereits beschrieben, können in dem erfindungsgemäßen Verfahren die untersuchten Analyten wie Sauerstoff und $CO_2$, Glukose und/oder der pH-Wert in der unmittelbaren Umgebung der 3D-Zellkulturen, also der Organoide bzw. Zellaggregate, gemessen werden. Außerdem ist es möglich, Gradienten der Analyte in der unmittelbaren Mikroumgebung der Zellaggregate bzw. Organoide zu messen. Parallel dazu ist bspw. die Messung von Calcium oder Calciumtransienten über fluoreszierende Proteine innerhalb der Zellen bzw. Organoide möglich.

Dadurch, dass das eingesetzte Messverfahren auf einer mikroskopischen Bestimmungsmethode basiert, sind zudem weitere Untersuchungen möglich, beispielsweise durch Vitalfärbungen der Zellen oder durch Änderungen in Gen- und Proteinexpression durch Einbringen geeigneter Sonden.

Die in dem erfindungsgemäßen Verfahren eingesetzten Assays basieren auf der Verwendung dreidimensionaler Strukturen, beispielsweise in Form von (Mikro)Kavitäten, die aus Sauerstoff-sensitiven funktionalisierten Sensorfolien geformt werden. Solche dreidimensionalen funktionalisierten Strukturen sind auch Gegenstand der unveröffentlichten Europäischen Patentanmeldung EP23180517.7 und können wie folgt näher beschrieben werden. Der Begriff "dreidimensionale Strukturen" bezeichnet hierin funktionalisierte, Sauerstoff-sensitive Sensorfolien, welche in eine dreidimensionale Form überführt (geformt) wurden, insbesondere in Form "dreidimensionaler Mikrostruktur(en)", und solche dreidimensionalen Strukturen sind Formen bzw. Formkörper, die eine Vertiefung, Höhlung, einen Hohlraum oder eine sonstige Form zur Aufnahme eines Volumens ausbilden. Im Prinzip können sie jede geometrische Form aufweisen, bevorzugt sind diese rund bzw. halbrund, kugelig oder weisen die Form von langgezogenen Kanälen, jeweils vorzugsweise mit nach außen gewölbtem Boden, auf. Dadurch sind diese dreidimensionalen Strukturen zur Aufnahme und/oder Kultivierung von Zellen geeignet und weisen daher eine Öffnung auf, die die Aufnahme von Zellen, Kulturmedien, sowie sonstiger Reagenzien zur Zellkultur und -analyse etc. ermöglicht. Je nach Größe der Strukturen können diese auch als sogenannte Mikrostrukturen oder Mikrokavitäten vorliegen, beispielsweise in Form von Mikrokavitätenarrays, Mikrokanälen oder anderen Mikrovertiefungen, und liegen damit insbesondere in bzw. als Mikrotiterplatten, Zellkulturplatten oder in Zellkulturinserts mit einem oder mehreren Kompartimenten vor. Besonders geeignet sind Mikrokavitäten, welche in Form runder bzw. halbrunder oder kanalförmiger Vertiefungen in der Sensorfolie ausgebildet sind. Solche runden oder halbrunden Vertiefungen können außerdem im oberen Bereich eine Fase aufweisen, wie nachfolgend detaillierter beschrieben. Bezüglich der Dimensionen der erfindungsgemäßen Kavitäten bezeichnet "Tiefe" den längsten Abstand vom Boden einer Kavität bis zu deren Rand ohne Fase (T2 in Fig. 5A bzw. T4 in Fig. 5B). Runde/halbrunde Kavitäten können durch ihren Durchmesser an der oberen Öffnung ohne Fase (D1 in Fig. 5A) charakterisiert werden, wohingegen bei kanalförmigen Kavitäten die "Breite" den kürzeren Abstand zwischen zwei Seitenwänden ohne Fase (B4 in Fig. 5B) im Gegensatz zu deren "Länge" mit dem längsten Abstand zwischen zwei Seitenwänden ohne Fase (L3 in Fig. 5B) bezeichnet.

Runde/halbrunde Kavitäten können durch ihren Durchmesser an der oberen Öffnung charakterisiert werden, wohingegen bei kanalförmigen Kavitäten die "Breite" den kürzeren Abstand zwischen zwei Seitenwänden im Gegensatz zu deren "Länge" mit dem längsten Abstand zwischen zwei Seitenwänden bezeichnet. Je nach Dimensionierung der Kavitäten handelt es sich dann um Kavitäten oder Mikrokavitäten. Die einzelnen (Mikro)Kavitäten der erfindungsgemäß eingesetzten dreidimensionalen Strukturen können einen Durchmesser (gemessen am oberen Rand der Kavität ohne Fase) bis 4.000 $\mu$m, bevorzugt bis 2.000 $\mu$m, bevorzugter bis 1.000 $\mu$m, noch bevorzugter bis 800 $\mu$m, noch bevorzugter bis 500 $\mu$m aufweisen. Die einzelnen (Mikro) Kavitäten der erfindungsgemäß eingesetzten dreidimensionalen Strukturen können einen Durchmesser (gemessen am oberen Rand der Kavität ohne Fase) von mindestens 10 $\mu$m, bevorzugt mindestens 50 $\mu$m, bevorzugter mindestens 100 $\mu$m, noch bevorzugter mindestens 200 $\mu$m, noch bevorzugter mindestens 300 $\mu$m aufweisen. Durchmesser im Bereich von 1 bis 4.000 $\mu$m, bevorzugt 10 bis 2.000 $\mu$m, bevorzugter 100 bis 1.000 $\mu$m, noch bevorzugter 200 bis 800 $\mu$m, noch bevorzugter 300 bis 500 $\mu$m sind möglich. Bei Verwendung von dreidimensionalen Strukturen in Form kanalförmiger Strukturen können diese eine Kanallänge (gemessen in der Kavität ohne Fase) von 120 mm, bevorzugt 60 mm, bevorzugter 20 mm aufweisen. Sie können eine Kanalbreite (gemessen in der Kavität ohne Fase) bis 4.000 $\mu$m, bevorzugt bis 2.000 $\mu$m, bevorzugter bis 1.000 $\mu$m, noch bevorzugter bis 800 $\mu$m, noch bevorzugter bis 500 $\mu$m aufweisen. Sie können eine Kanalbreite (gemessen in der Kavität ohne Fase) von mindestens 10 $\mu$m, bevorzugt mindestens 50 $\mu$m, bevorzugter mindestens 100 $\mu$m, noch bevorzugter mindestens 200 $\mu$m, noch bevorzugter mindestens 300 $\mu$m aufweisen. Kanalbreiten im Bereich von 1 bis 4.000 $\mu$m, bevorzugt 10 bis 2.000 $\mu$m, bevorzugter 100 bis 1.000 $\mu$m, noch bevorzugter 200 bis 800 $\mu$m, noch bevorzugter 300 bis 500 $\mu$m sind möglich.

Insbesondere zur Bestimmung von Analytgradienten ist die Ausbildung einer sogenannten Fase an der Öffnung im oberen Bereich der Kavitätenstrukturen vorteilhaft. Durch die zusätzliche Ausbildung einer solchen Fase am oberen Rand der Kavität wird eine Art Rampe oder schiefe Ebene in die Struktur integriert, an welcher in der zweidimensionalen Aufnahme durch Messung an verschiedenen Punkten Informationen entlang der Fase und somit zu unterschiedlichen Tiefen gewonnen werden können. In Figur 5 werden die Dimensionen der Fase durch einen Durchmesser bzw. (in kanalförmigen Kavitäten) durch eine Breite D2 bzw. B3, jeweils gemessen am obersten Rand der Fase, sowie durch eine Höhe der Fase T2 bzw. T3, jeweils gemessen vom oberen Rand der Kavität bis zum oberen Rand der Fase, illustriert.

Solche Fasen weisen bevorzugt einen Öffnungswinkel von 1 bis 179°, bevorzugt von 20 bis 120°, noch bevorzugter von 30 bis 60° auf. Figur 5A und 5B illustrieren diesen Öffnungswinkel $\alpha$.

Sind mehrere solcher dreidimensionaler Strukturen in Nachbarschaft zueinander auf einer Fläche angeordnet, so dass mehrere Kavitäten oder kanalförmige Strukturen mit gleichen oder unterschiedlichen Formen und/oder Dimensionen (d.h. gleichen oder unterschiedlichen Längen, Breiten, Durchmessern, Tiefen und runden bzw. kanalförmigen Formen) nebeneinander und auf einer Fläche, z.B. einer Fläche von 120 x 80 mm², vorliegen, so bilden diese eine sogenannte Multikavitäten-Struktur aus. Liegen in derartigen Multikavitäten-Strukturen die einzelnen Kavitäten in Form kanalförmiger Kavitäten vor, so können diese grundsätzlich geradlinig, verzweigt oder mäanderförmig angeordnet sein. Die Tiefe der dreidimensionalen Strukturen kann durch das As-

pektverhältnis (Durchmesser Tiefe) der einzelnen Kavität charakterisiert werden, wobei das Aspektverhältnis der einzelnen Kavitäten bevorzugt 1:2, bevorzugter 1:1, noch bevorzugter 2:1 beträgt. Die einzelnen (Mikro)Kavitäten der eingesetzten dreidimensionalen Strukturen können eine Gesamttiefe bis 800 μm, bevorzugt bis 700 μm, bevorzugter bis 600 μm, noch bevorzugter bis 500 μm, noch bevorzugter bis 450 μm und/oder eine Gesamttiefe von mindestens 100 μm, bevorzugt mindestens 200 μm, bevorzugter mindestens 250 μm aufweisen, wobei eine Gesamttiefe im Bereich von 100 bis 600 μm, bevorzugt 200 bis 500 μm, bevorzugter 250 bis 450 μm realisiert werden kann. Besonders geeignet sind dreidimensionale Strukturen, welche runde Kavitäten aufweisen und die sich wie folgt charakterisieren lassen: Die Form der einzelnen Kavität (1) entspricht einer gerundeten, geöffneten Form, beispielsweise einer Halbkugel oder (bei größeren Durchmessern) einer Form, die flacher als eine Halbkugel ist oder (bei kleineren Durchmessern) einer Form, die nach der Halbkugel noch einen senkrechten Bereich aufweiset. Die Tiefe der einzelnen Kavität (T1) ist im Optimalfall $T1 = \frac{D1}{2}$, wobei generell $0 < T1 \leq D1$ möglich ist. Noch tiefere Kavitäten sind denkbar, aber für Zellkulturanwendungen eher unrelevant. Für den Durchmesser (D2) der Fase (5) gilt D2 > D1, für die Tiefe (T2) gilt T2 > 0. Aus D2 und T2 ergibt sich dann auch der Öffnungswinkel α. Größere Durchmesser für D1 als 4000 μm sind technisch möglich, für den Bereich der Zellkultur aber nur bedingt relevant, zudem würde man hier dann eher von Makrokavitäten sprechen. Durchmesser von bis zu 1 μm sind mit dünneren Folien denkbar, aber nur für Anwendungen außerhalb der Zellkultur sinnvoll.

Werden kanalförmige Kavitäten eingesetzt, optional auch mit Fase, lassen sich besonders geeignete Ausführungsformen wie folgt charakterisieren:

Die Größenordnungen sind vergleichbar mit denen der vorstehend beschriebenen bevorzugten runden Kavitäten, wobei statt des Durchmessers die Breite des Kanals (B4) definiert wird, welche 10 bis 4000 μm betragen kann. Da der Kanalquerschnitt im Optimalfall halbrund ist, gilt für die Tiefe $T4 = \frac{D4}{2}$, wobei generell $0 < T4 \leq D4$ möglich ist. Die Fase kann wie bei der runden Kavität gestaltet sein. Kanallängen (L3) liegen üblicherweise im Bereich bis zu 40 mm, längere und auch verzweigte Kanäle sind denkbar und möglich.

Die Mikrokavitäten können einzeln vorliegen oder in Mikrokavitätenarrays angeordnet sein, wobei sich darin die einzelnen Kavitäten berühren oder auch über einen Abstand verfügen können. Ein einzelnes Array verfügt über mehrere Mikrokavitäten, die in Spalten und Zeilen mit verschiedener Anzahl angeordnet sein können. Üblicherweise werden Arrays mit 30 bis 1000 Mikrokavitäten verwendet. Solche Anordnungen können sich auch auf Folien im Format einer Multiwellplatte erstrecken, sodass in einem Abformvorgang je Folie bis zu 20.000 Mikrokavitäten generiert werden können. Derartige Multikavitäten-Strukturen können bis zu 1.000.000 Mikrokavitäten pro cm$^2$ aufweisen, bevorzugt weisen solche Multikavitätenstrukturen mindestens 1 bis 1.000.000 Mikrokavitäten/cm$^2$ auf, bevorzugt zwischen 10 und 10.000 Mikrokavitäten/cm$^2$, noch bevorzugter zwischen 100 und 1000 Mikrokavitäten/cm$^2$. Diese können dabei in gerade Linie, parallel nebeneinander oder zueinander versetzt angeordnet sein. Die Multikavitätenstrukturen können über die gesamte Fläche gleichmäßig verteilt vorliegen oder nur in bestimmten Bereichen vorgesehen werden und z.B. als "Gruppen" organisiert angeordnet werden. Eine solche beispielhafte gruppierte Anordnung illustriert die Figur 4.

Da die erfindungsgemäß eingesetzten dreidimensionalen Strukturen aus funktionalisierten Sensorfolien geformt werden, handelt es sich im Grunde genommen bei diesen um (mikro)strukturierte funktionalisierte Sensorfolien, die aufgrund der Verformung zu dreidimensionalen Strukturen zur kombinierten Zellkultur und Analytmessung geeignet sind. Dabei dient die "Messvorrichtung", nämlich die strukturierte (geformte) Sensorfolie, selbst auch als Kultivierungsmaterial. Funktionalisierte Sensorfolien, die zur Bildung / Formung der dreidimensionalen Strukturen verwendet werden, sind solche Folien worin ein geeigneter Träger derart dotiert, beschichtet oder in sonstiger Weise behandelt wurde, dass eine Funktionalisierung erfolgt mittels derer gezielt die analytische Messung oder Bestimmung der Parameter möglich ist für die sie eingesetzt werden. Üblicherweise umfassen solche funktionalisierten Sensorfolien eine Trägerfolie, z.B. in Form von Polymerträgerfolien, welche üblicherweise eine Dicke zwischen 1 μm und 100 μm, bevorzugter bis 50 μm aufweisen. Für das Verfahren der vorliegenden Erfindung sind die dreidimensionalen Strukturen aus funktionalisierten Sauerstoff-sensitiven Sensorfolien gebildet. Entsprechend geeignete funktionalisierte Sauerstoff-sensitive Sensorfolien umfassen fluoreszierende Folien, wie Fluorophor-dotierte Folien. Die Dotierung kann grundsätzlich in allen üblichen Formen erfolgen und beispielsweise in Form einer Beschichtung vorliegen. Es ist ebenfalls möglich, dass die Fluorophore in Hydrogelen oder in Polymermatrizes immobilisiert und in dieser Form auf die Trägerfolie aufgebracht sind. Ein besonderer Vorteil der eingesetzten dreidimensionalen Strukturen liegt darin, dass die Funktionalisierung der Sensorfolien Hydrogel-frei und ohne Verwendung von Nanopartikel-gebundenen Fluorophoren erfolgen kann, wobei klarstellend anzumerken ist, dass "Hydrogel-frei" bzw. "ohne Verwendung von Nanopartikel-gebundenen Fluorophoren" nicht solche geformten Sensorfolien ausschließt, worin die Fluorophore über solche Mittel in der Sensorfolie dotiert (gebunden) vorliegen.

Fluorophor-dotierte Sensorfolien umfassen beispielsweise solche, worin die Fluorophore ausgewählt sind aus der Gruppe umfassend Metallkomplexe, insbesondere solche aus der Gruppe der Platin-II-Porphyrine, Palladium-II-Porphyrine, Ruthenium-II-Komplexe, Platin-/Palladium-Komplexe sowie Iridium- und Europium-Komplexe. Je nach Sensitivität können unterschiedliche Fluorophore auf unterschiedliche Analyten ansprechen, welche zur gleichen oder zu unterschiedlichen Gruppen von Metallkomplexen gehören können.

Die für das erfindungsgemäße Verfahren eingesetzten Assays bzw. Messaufbauten umfassen vorteilhafterweise ein oder mehrere Messinstrumente, die dazu verwendet werden, die zu untersuchenden Analyten und/oder Analytgradienten zu detektieren und zu erfassen. Die Datenerfassung ist idealerweise automatisiert. Dazu werden die Mess- und Detektionseinheiten Computer-gesteuert betrieben und umfassen vorzugsweise eine geeignete Auswertungssoftware, sodass die erfassten Messwerte sofort verarbeitet, ausgewertet und ausgegeben werden können.

Hinsichtlich der Auswertung kann auch ein KI-System integriert werden, welches einen Abgleich der gemessenen Daten mit bereits vorhandenen Daten aus einer Datenbank ausführt und Ergebnisinterpretation anbietet. Dies ermöglicht es schnell und zielsicher aussagekräftige Ergebnisse zu erlangen. Werden über eine KI-Integration anhand von bereits zur Verfügung stehender Daten die gemessenen Ergebnisse in Bezug gesetzt, so können beispielsweise Toxizitäts- oder Nebenwirkungsprofile abgeschätzt werden, indem die erhaltenen Messwerte mit bereits bestehenden Messwerten, die bekannte Korrelationen zu den untersuchten Schadwirkungen aufweisen, abgeglichen werden.

Die Messanordnung für die eingesetzten Assays kann in Form fluidischer Systeme oder in Form von Mikrobioreaktoren eingesetzt werden, welche die hierin beschriebenen dreidimensionalen Strukturen der funktionalisierten Sensorfolien umfassen. Solche fluidischen Systeme und Mikrobioreaktoren sowie die Herstellung solcher Systeme ist in der unveröffentlichten Europäischen Patentanmeldung EP23180517.7 beschrieben, welche unter Bezugnahme vollumfänglich von der vorliegenden Erfindung umfasst wird.

Wie bereits beschrieben kann mit dem hierin beschriebenen Assay das erfindungsgemäße Verfahren zur Bestimmung von Sauerstoff und Sauerstoffgradienten in 3D-Zellkulturen von Kardiomyozytenaggregaten oder Herzorganoiden durchgeführt werden, ggf. in Kombination (z.B. unter paralleler oder simultaner Bestimmung) mit der Bestimmung weiterer relevanter Analyten bzw. Analytgradienten, um Ergebnisse zur Kardiotoxizität oder ischämischen Toxikologie zu erhalten. Untersuchungen zu Kardiotoxizität oder ischämischer Toxikologie sind insbesondere von Interesse, um die Auswirkungen von Testsubstanzen, wie Wirkstoffen oder Chemikalien, z.B. auf mitochondriale Atmungsprozesse, zu untersuchen. Durch das erfindungsgemäße Verfahren ist es möglich, solche Untersuchungen *in vitro* in einer physiologisch betrachtet realistischen Umgebung durchzuführen.

Durch die erfindungsgemäß bevorzugte Kombination der Bestimmung von Sauerstoff und der Untersuchung der Calciumtransienten in einer 3D-Zellkultur von Kardiomyozytenaggregaten oder von Herzorganoiden können genauere Voraussagen über mögliche Substanz-induzierte Einflüsse der getesteten Verbindungen, wie von Medikamenten oder sonstigen Wirkstoffen oder allgemein Chemikalien, getroffen werden. So können bereits im *in* vitro-Modell Einschätzungen mit hoher Aussagekraft zur Kardiotoxizität einer chemischen Substanz ermittelt werden.

Zur Ermittlung der ischämischen Toxizität oder Kardiotoxizität können in dem erfindungsgemäßen Verfahren die eingesetzten Zellen zunächst einem Stress-Test, z.B. einem Mito-Stress-Test, unterworfen werden. Dies kann in bekannter Weise, beispielsweise durch die schrittweise Zugabe von Stress-induzierenden Testsubstanzen wie Oligomycin, FCCP bzw. Rotenon und Antimycin A erfolgen. Durch die Bestimmung der hierin beschriebenen Analyten können dann mit dem erfindungsgemäßen Verfahren Aussagen über die Basalatmung, die ATP-basierte Atmung, sowie den maximalen Sauerstoffumsatz getroffen werden und Aussagen über den Einfluss der Testsubstanzen auf die Atmungskette getroffen werden. Zur Untersuchung welche Komplexe der Atmungskette beeinflusst werden ist beispielsweise auch ein Mito-Complex-Assay in dem erfindungsgemäßen Verfahren einsetzbar.

Dies ist grundsätzlich für die chemische und pharmazeutische Industrie, aber beispielsweise auch in der Entwicklung von Wirkstoffkosmetik, von großem Interesse. Für die pharmazeutische oder kosmetische Industrie ermöglicht das erfindungsgemäße Verfahren frühzeitig und auf einfache Weise (Neben)Wirkungsprofile von Wirkstoffen oder Wirkstoffkandidaten zu erhalten, ohne dafür auf Tierversuche zurückgreifen zu müssen. Aber auch die Klassifizierung von Gefahrstoffen nach dem REACH (Registration, Evaluation, Authorisation of Chemicals) System wird dabei ohne ethisch problematische Experimente ermöglicht. Entsprechend des REACH-Systems sind Unternehmen, die mit Chemikalien handeln dazu verpflichtet, diese in ausreichender Weise hinsichtlich ihres Gefahrenpotenzials zu charakterisieren. Das erfindungsgemäße Verfahren kann auch hier verwendet werden, um solche möglichen toxikologischen Gefahren zu untersuchen.

Die Erfindung umfasst somit auch einen Assay, basierend auf dem hierin beschriebenen Verfahren, zur Untersuchung der Auswirkungen von Testsubstanzen auf den Calciumstoffwechsel (Calciumtransienten), sowie einen Assay zur Messung der ischämischen Toxizität, basierend auf dem hierin beschriebenen Verfahren.

## BESCHREIBUNG DER FIGUREN

Fig. 1    (A) Schematische Darstellung einer zweidimensionalen Zellkultur auf kommerziell erhältlichen planaren Sauerstoff-sensitiven Folien, und

(B) Schematische Darstellung der Generierung einer dreidimensionalen Zellkultur in Sauerstoff-sensitiven Mikrokavitätenarrays, zur Bestimmung der Sauerstoffkonzentrationen in unmittelbarer Mikroumgebung der Zellen gemäß dem erfindungsgemäßen Verfahren.

Fig. 2     (A) Schematische Darstellung von Strukturen gemäß Stand der Technik unter Verwendung einer mit einer planaren Sensorfolie beschichteten Rampe als Insert auf dem Boden einer 2D-Zellkultur zur Messung von Sauerstoffgradienten

(B) Schematische Darstellung von Strukturen gemäß Stand der Technik unter Verwendung einer mit einer planaren Sensorfolie beschichteten Rampe als Insert eingehängt in eine 2D-Zellkultur zur Messung von Sauerstoffgradienten.

(C) Schematische Darstellung von Strukturen gemäß Stand der Technik unter Verwendung einer mit einer planaren Sensorfolie beschichteten Rampe als Insert eingehängt in eine 3D-Zellkultur zur Messung von Sauerstoffgradienten.

(D) Schematische Darstellung von erfindungsgemäßen Mikrokavitäten mit Fase am Boden eines Zellkulturinserts zur Messung von Sauerstoffkonzentrationen/-gradienten in kugelförmigen 3D-Zellkulturen.
(E) Schematische Darstellung von erfindungsgemäßen Mikrokavitäten mit Fase als Boden der Wells einer Mikrotiterplatte zur Messung von Sauerstoffkonzentrationen/-gradienten in kugelförmigen 3D-Zellkulturen.

Fig. 3     Schematische Darstellung des Messprinzips zur Bestimmung der ischämischen Toxizität, welche bisher in separaten Verfahren erfolgte ((A) die Mikroskopie, (B) Sauerstoffmessung) und welche im erfindungsgemäßen Verfahren in der Mikroumgebung von 3D-Zellaggregaten möglich ist, indem die Sauerstoffmessung und die mikroskopische Auswertung in einem System (Messansatz) möglich sind (C). Durch Verwendung von Zellen mit einem internen Calciumsensor sind zusätzlich Calciumtransienten messbar und durch kombinierte Auswertung aus Sauerstoffkonzentrationen (bzw.

dem Sauerstoffverbrauch) und den Calciumtransienten werden komplexere Untersuchungen zu Substanz-induzierten Veränderungen möglich. Hierbei können verschiedene ROI (= region of interest) betrachtet werden (D), um den Einfluss einer Testsubstanz sowohl auf die Sauerstoffkonzentration als auch bspw. auf die Schlagfrequenz zu zeigen (E).

Fig. 4     (A) Zellkulturinsert mit 4 Kompartimenten (Wells);

(B) Schematische Zeichnung der Aufteilung der Wells und der Mikrokavitäten;

(C) Skizze einer möglichen Anordnung der Mikrokavitäten.

(D) Positionierung möglicher Sensorspots im Zellkulturinsert, entweder direkt auf der Folie mit den Mikrokavitäten oder als Teil des Zellkulturinserts (schraffiert).

Fig. 5     (A) Schematische Darstellung einer runden Kavität mit Fase im Querschnitt.

(B) Schematische Darstellung einer kanalförmigen Kavität mit Fase im Querschnitt.

(C) Schematische Darstellung einer runden Kavität mit Fase in der Aufsicht.

(D) Schematische Darstellung einer kanalförmigen Kavität mit Fase in der Aufsicht.

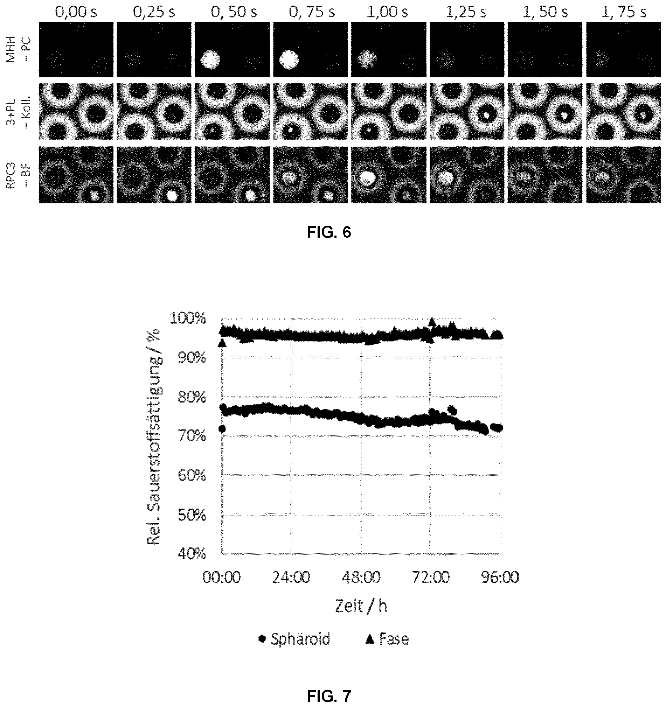

Fig. 6     Zeitserie des intrazellulären Calciumsignals, gemessen mit einem GFP-Filterset. Dargestellt sind Kardiomyozytenaggregate aus dem Bioreaktor in PC-Mikrokavitäten (MHH-PC), sowie in den Mikrokavitäten generierte Aggregate in Sensorarrays mit Plasmabehandlung und Kollagenisierung (3+PL-Koll) sowie in BIOFLOAT™ - beschichteten Sensorarrays (RPC3-BF). Gesamtvergrößerung: 100 x.

Fig. 7:     Sauerstoffmessung an Kardiomyozytenaggregaten aus humanen induzierten pluripotenten Stammzellen in Sensorarrays im Bereich des Sphäroids sowie im Bereich der Fase über 96 h.

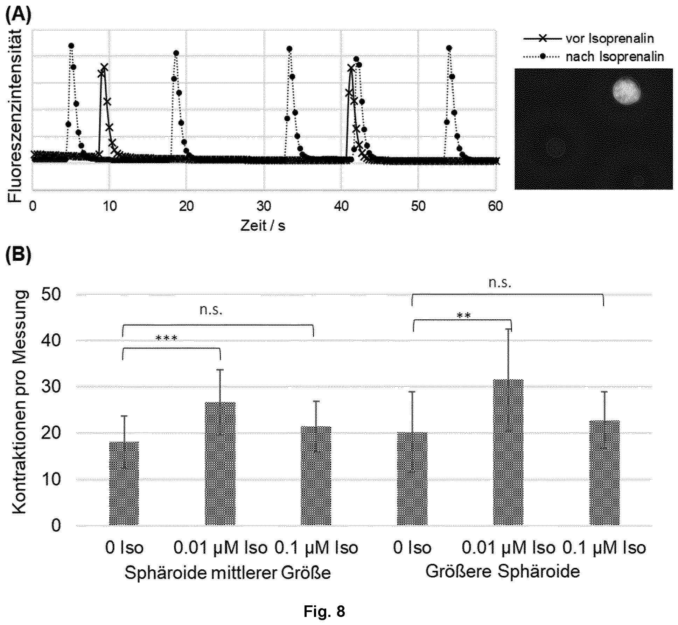

Fig. 8     Kontraktionen vor und nach der Behandlung mit Isoprenalin. Die Kontraktionen wurden durch die Messung des Calciumsignals bestimmt. Die Signifikanz wurde mithilfe eines zweiseitigen Mann-Whitney-U-Tests berechnet. n.s.: nicht signifikant, **: signifikant (Konfidenzintervall 97,5 %), ***: signifikant (Konfidenzintervall: 99 %), Iso: Isoprenalin.

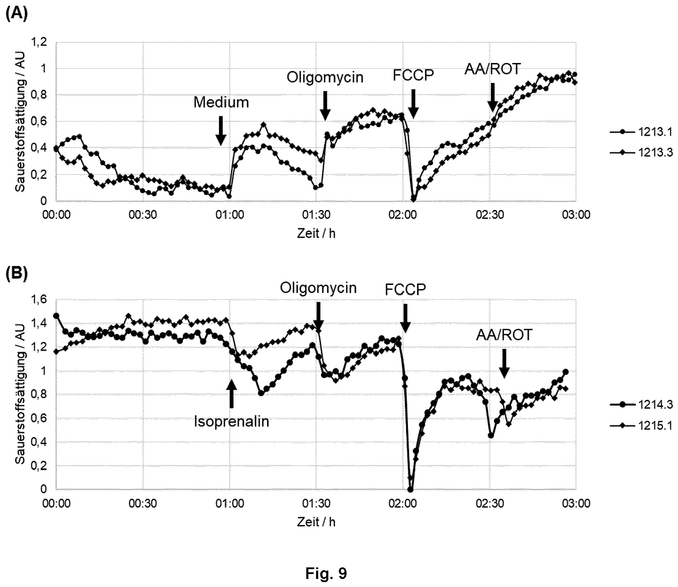

Fig. 9 (A) Darstellung der Sauerstoffsättigung während des Mito-Stress-Tests an Kardiomyozytenaggregaten (dargestellt sind zwei unabhängige Versuche).

  (B) Einfluss von 0,01 (hellgrau) bzw. 0,04 μM Isoprenalin während des Mito-Stress-Tests.

**BEZUGSZEICHEN**

(1) Dreidimensionale Struktur(en) (Mikrostrukturen / Mikrokavität(en))
(2) Mikrokavitätenarrays / Mikrotiterplatten / Wellplatten / Well
(3) Zellkulturinsert
(4) funktionalisierte Sensorfolie
(5) Fase(n)
(6) Zellen / (3D-)Zellkultur / Sphäroid(e)
(7) Detektionselement / Mikroskop
(8) Rampe
(9) Funktionalisierungsspot / Sensorspot

**BEISPIELE**

**Untersuchung der ischämischen Toxizität an aus pluripotenten Stammzellen abgeleiteten Kardiomyozyten**

Aus induzierten, pluripotenten Stammzellen abgeleitete Kardiomyozyten [*Drakhlis et al. 2021; Drakhlis, Devadas, and Zweigerdt 2021; Halloin et al. 2019*] werden in Mikrokavitätenarrays in einem Zellkulturinsert oder einer Mikrotiterplatte mit Mikrokavitätenarrays kultiviert. Die Herstellung kann entsprechend dem in EP3765599B1 beschriebenen Herstellverfahren direkt in den Mikrokavitätenarrays erfolgen. Durch die Beschichtung der Mikrokavitäten mit BIOFLOAT™ (faCellitate GmbH) wird die Bildung von Sphäroiden direkt in den Mikrokavitäten unterstützt, so dass sich in den beschichteten Kavitäten kugelige Aggregate (Sphäroide) der kultivierten Kardiomyozyten bilden.

Bei den eingesetzten Mikrokavitätenarrays handelt es sich um solche, die durch Beschichtung einer Polymerfolie mit einem Sauerstoff-sensitiven Fluorophor gemäß Herstellbeispiel 3 der unveröffentlichten Europäischen Patentanmeldung EP23180517.7 mittels Mikrothermoformverfahren hergestellt wurden, indem zunächst eine Formmaske (Negativ-Maske) aus Messing gefertigt wurde, welche an der Öffnung zu den Kavitäten jeweils eine Fase umfassen. In der Formmaske wurde die Sauerstoff-sensitive Folie die im Öffnungsbereich über eine Fase zur Bestimmung von Gradienten verfügen, abgeformt.

Die Sauerstoffkonzentration kann bereits während der Kultivierungsphase bestimmt und untersucht werden. Die Sauerstoffkonzentration kann im Bereich der Fase der Mikrokavitäten mittels Mikroskopie bspw. mit einem VisiSens-System der Firma PreSens Precision Sensing GmbH oder anderen Konfokal- oder Fluoreszenzmikroskopen in der Sphäroidumgebung gemessen werden.

Zur Charakterisierung der mitochondrialen Atmung werden die Zellen zunächst einem Mito-Stress-Test unterzogen, wie im Detail in [Christoph Grün, Jana Pfeifer, Gregor Liebsch, Eric Gottwald: O2-sensitive microcavity arrays: A new platform for oxygen measurements in 3D cell cultures. Frontiers in Bioengineering and Biotechnology 2023, DOI: 10.3389/fbioe.2023.1111316] beschrieben. Zudem kann im Rahmen eines solchen Mito-Stress-Tests die Langzeit-Toxizität unbekannter Substanzen ermittelt werden.

Zur Bestimmung einer maximalen und minimalen Sauerstoffkonzentration in den Kavitäten wird zunächst eine Kalibrierung auf den Mikrokavitätenarrays ausgeführt. Dazu wird zum einen die Sauerstoffkonzentration in einem zuvor 10 min unter den Kultivierungsbedingungen equilibrierten 100 μL Wassertropfen gemessen, um die maximale Sauerstoffkonzentration zu bestimmen. Zum anderen wurde dieser Vorgang mit einer 10 mg/ml Natriumsulfit-Lösung zur Bestimmung der minimalen Sauerstoffkonzentration wiederholt.

Die Sauerstoffkonzentration an den ausgewählten Stellen der Sphäroidumgebung wird in einem ersten Schritt für 30 min gemessen, um die Einstellung eines Gleichgewichtes in der Sauerstoffkonzentration zu erfassen. In einem zweiten Schritt wird nach 30 min erneut Kulturmedium zugesetzt und die Sauerstoffkonzentration weiter gemessen, um die basale Zellatmung zu bestimmen. In einem dritten Schritt wird nach weiteren 30 min schrittweise Oligomycin, sowie in einem vierten Schritt nach weiteren 30 min Carbonylcyanid-4-(trifluormethoxy)phenylhydrazon (FCCP) zugesetzt und die Sauerstoffkonzentration weiter gemessen, um die maximale Zellatmung zu erfassen. In einem fünften Schritt wird nach weiteren 30 min Rotenon und Antimycin A zugesetzt und die Sauerstoffkonzentration weiter gemessen, um die nicht-mitochondriale Zellatmung zu erfassen.

Aus den erhaltenen Daten können Aussagen über die Basalatmung, die ATP-basierte Atmung, sowie den maximalen Sauerstoffumsatz getroffen werden. Nach der Bestimmung der basalen Atmungsparameter, können diese dann nach Substanzgabe erneut gemessen werden. Durch Bestimmung der Sauerstoffkonzentration im Kulturmedium in Abhängigkeit von der Zeit und Zugabe der untersuchten Testsubstanzen sind Aussagen über den Einfluss der untersuchten Testsubstanz auf die Atmungskette möglich. Eine steigende Sauerstoffkonzentration spricht für eine inhibierte Zellatmung durch die Testsubstanz, da weniger Sauerstoff aus der Umgebung durch die Sphäroide verbraucht wird.

In diesem Mito-Stress-Test wird die Sauerstoffkonzentration an ausgewählten Positionen in der Sphäroidumgebung unter verschiedenen Bedingungen untersucht. Messungen werden in Wiederholungsintervallen ausgeführt. Dadurch wird ein Aufschluss über das Zellatmungsverhalten unter den untersuchten Gegebenheiten erlangt.

Unterschiedliche Messkurven für unterschiedliche Positionen in der Sphäroidumgebung verdeutlichen die dreidimensionale Ortsabhängigkeit des Sauerstoffverbrauchs um die Sphäroide.

Parallel zur Sauerstoffmessung kann der Einfluss auf die Schlagfrequenz fluoreszenzmikroskopisch bestimmt werden.

Dazu erfolgt eine Bestimmung der Calcium-Transienten über die fluoreszenmikroskopische Detektion der Bindung von Calcium an in die Zellen eingeschleuste GECIs. Die zeitliche Auflösung solcher Aufnahmen lässt einen Rückschluss auf die dabei auftretenden Transienten zu. Figur 6 zeigt eine solche Calcium-Transienten Detektion, worin das Calcium-Bild mit der Zeit heller und dann wieder dunkler wird. Dies entspricht einer Zunahme bzw. Abnahme der Calciumkonzentration und damit den Calcium-Transienten.

Die Messungen der Sauerstoffkonzentrationen, sowie der Schlagfrequenzen erfolgte im Wechsel im selben Zellkulturmodell. Durch Integration des Fluoreszenzmikroskops in das System zur Sauerstoffmessung ist auch eine simultane Messung möglich. Es konnte gezeigt werden, dass verschiedene Testsubstanzen einen Einfluss auf die Schlagfrequenz der Aggregate haben.

Durch Einsatz von Mikrokavitätenarrays, welche mit zusätzlichen Funktionalisierungsspots ausgestattet sind, kann außerdem die $CO_2$- und/oder Glukose-Konzentration sowie der pH-Wert in der Kultur untersucht werden.

Für entsprechende Untersuchungen wurden Stammzell-abgeleitete Kardiomyozytenaggregate in den erfindungsgemäßen Sauerstoff-sensitiven Mikrokavitäten untersucht. Zum einen erfolgte die Aggregation direkt in den Mikrokavitäten, zum anderen aber vorab in einem Bioreaktor. Zunächst konnte in den Mikrokavitäten die Schlagfrequenz gemessen werden (siehe Fig. 6). Die Sauerstoffkonzentration wurde mehrere Tage parallel dazu bestimmt (Fig. 7). Das Sympathomimetikum Isoprenalin erhöht die Schlagfrequenz des Herzens, was sich durch einen erhöhten Calciumeinstrom in die Herzmuskelzellen zeigt. In dem hier beschriebenen Messsystem konnte die Erhöhung der Schlagfrequenz durch Messung der Fluoreszenz der GECI gezeigt werden (Fig. 8). Der Einfluss von Isoprenalin zeigte sich auch in einem 3D-Mito-Stress-Test durch eine Abnahme der Sauerstoffsättigung im Vergleich zur Mediumkontrolle, was auf eine erhöhte Aktivität der Zellen schließen lässt (Fig. 9).

Zur Untersuchung welche Komplexe der Atmungskette beeinflusst werden ist auch ein Mito-Complex-Assay in diesem System durchführbar und wurde getestet.

Der hierin beschriebene Assay kann analog zur Untersuchung des Einflusses bzw. die Wechselwirkungen mehrerer parallel verabreichter Testsubstanzen eingesetzt werden.

## Patentansprüche

1. Verfahren zur *in* vitro-Messung von Sauerstoffkonzentrationen und/oder Sauerstoffkonzentrationsgradienten in 3D-Zellkulturen von Kardiomyozyten oder an Herzorganoiden (HFOs), umfassend

   a) Bereitstellung von dreidimensionalen Strukturen die aus Fluorophor-dotierten Sauerstoffsensitiven Sensorfolien geformt sind,
   b) Kultivierung der 3D-Zellkulturen in Form von Kardiomyozytenaggregaten oder Herzorganoiden in den dreidimensionalen Fluorophor-dotierten Sauerstoff-sensitiven Sensorfolien oder Überführung der bereits kultivierten 3D-Zellkulturen oder der Herzorganoide in die dreidimensionalen Fluorophor-dotierten Sauerstoff-sensitiven Sensorfolien,
   c) Sauerstoffmessung in der 3D-Zellkultur mittels optischer Methoden, wie der Mikroskopie,
   d) Erfassung und Auswertung der Messdaten.

2. Verfahren gemäß Anspruch [1], worin zusätzlich zur Messung der Sauerstoffkonzentration und/oder von Sauerstoffkonzentrationsgradienten die Messung von intrazellulären Calciumkonzentrationen und/oder -Transienten in derselben Zellkultur erfolgt.

3. Verfahren gemäß Anspruch [2], worin die Messung von intrazellulären Calciumkonzentrationen und/oder -Transienten über Genetically Engineered Calcium Indicators (GECI) erfolgt.

4. Verfahren gemäß einem der Ansprüche [1] bis [3], worin zusätzlich einer oder mehrere der Parameter $CO_2$, Glukose und pH-Wert in der 3D-Zellkultur gemessen werden.

5. Verfahren gemäß einem der Ansprüche [1] bis [4], worin es sich bei den dreidimensionalen Strukturen, die aus Fluorophor-dotierten Sauerstoff-sensitiven Sensorfolien gebildet sind, um Mikrokavitäten oder Mikrostrukturen handelt, die zur Aufnahme und/oder Kultivierung von Zellen geeignet sind und welche zusätzliche Funktionalisierungen mit $CO_2$-, Glukose- und/oder pH-Sensitivität aufweisen können.

**6.** Verfahren gemäß einem der Ansprüche [1] bis [5], worin die 3D-Zellkulturen von Kardiomyozyten oder die Herzorganoide (HFOs) aus pluripotenten, omnipotenten oder multipotenten Stammzellen, bevorzugt aus humanen pluripotenten, omnipotenten oder multipotenten Stammzellen, bevorzugter aus humanen, induzierten, pluripotenten Stammzellen differenziert oder aus primären Kardiomyozyten generiert werden.

**7.** Verfahren gemäß einem der Ansprüche [1] bis [6], worin die *in* vitro-Messung der Analyten an Herzorganoiden (HFOs) erfolgt.

**8.** Verfahren gemäß einem der Ansprüche [1] bis [7], worin sowohl die Kultivierung der 3D-Zellkultur oder der Herzorganoide als auch die Messung der Analyten in den dreidimensionalen Strukturen erfolgt.

**9.** Verfahren gemäß einem der Ansprüche [1] bis [8], worin die Analytmessung an verschiedenen Messpunkten (ortsaufgelöst) unter Erfassung des Analytgradienten zur Bestimmung von Sauerstoff-, Calcium-, $CO_2$-, Glukose-Konzentrationen und/oder pH-Gradienten erfolgt.

**10.** Verfahren gemäß einem der Ansprüche [1] bis [9], worin die dreidimensionalen Strukturen, die aus Fluorophor-dotierten Sauerstoff-sensitiven Sensorfolien geformt sind, in Form von Mikrokavitätenarrays, Mikrokanälen oder anderen Mikrovertiefungen für die Anwendung (den Einsatz) in Mikrotiterplatten, in Zellkulturplatten oder in Zellkulturinserts mit einem oder mehreren Kompartimenten vorliegen.

**11.** Verfahren gemäß einem der Ansprüche [1] bis [10], worin die dreidimensionalen Strukturen, die aus Fluorophor-dotierten Sauerstoff-sensitiven Sensorfolien geformt sind, in Form von Mikrokavitäten vorliegen, worin die Kavitäten eine Fase an der Öffnung der Kavitäten aufweisen, worin die Fasen bevorzugt einen Öffnungswinkel von 1 bis 179°, bevorzugter von 20 bis 120°, noch bevorzugter von 30 bis 60° aufweisen.

**12.** Verfahren gemäß einem der Ansprüche [1] bis [11], worin die Fluorophore ausgewählt sind aus der Gruppe der Metallkomplexe, umfassend insbesondere Platin-II-, Palladium-II- und Ruthenium-II-Komplexe.

**13.** Verfahren gemäß einem der Ansprüche [1] bis [12], worin die dreidimensionalen Strukturen Bereiche mit zusätzlicher Funktionalisierung mit $CO_2$-, Glukose- und/oder pH-Sensitivität aufweisen, welche in den dreidimensionalen Strukturen räumlich getrennt von den Sauerstoff-sensitiven funktionalisierten Bereichen angeordnet sind.

**14.** Verfahren gemäß einem der Ansprüche [1] bis [13], worin die Erfassung und Auswertung der Messdaten mit einem oder mehreren Elementen zur automatisierten Erfassung von Messdaten, Datenverarbeitung, Datenauswertung und Datenausgabe und/oder mittels KI-gestützter Evaluation der Messdaten erfolgt.

**15.** Verwendung des Verfahrens gemäß einem der Ansprüche [1] bis [14] zur Untersuchung des Einflusses von Testsubstanzen auf die mitochondriale Atmung, zur Bestimmung der ischämischen Toxizität und/oder zur Bestimmung der Kardiotoxizität, insbesondere zur Klassifikation von Wirkstoffen und/oder Wirkstoffkandidaten in der pharmazeutischen oder kosmetischen Industrie oder zur Evaluation von Chemikalien nach dem REACH-(Registration, Evaluation, Authorisation of Chemicals) System.

# FIGUREN

**FIG. 1**

**FIG. 2**

FIG. 3

FIG. 4

FIG. 5

FIG. 6

FIG. 7

**(A)**

**(B)**

Fig. 8

**(A)**

**(B)**

Fig. 9

Europäisches
Patentamt

European
Patent Office

Office européen
des brevets

# EUROPÄISCHER RECHERCHENBERICHT

**Nummer der Anmeldung**

EP 23 21 6372

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | CARLOS J. PENICHE SILVA: "A New Non-invasive Technique for Measuring 3D-Oxygen Gradients in Wells During Mammalian Cell Culture", FRONTIERS IN BIOENGINEERING AND BIOTECHNOLOGY, Bd. 8, 17. Juni 2020 (2020-06-17), XP093153530, CH ISSN: 2296-4185, DOI: 10.3389/fbioe.2020.00595 ----- | 1-15 | INV. G01N33/50 C12M1/12 C12M1/00 C12M1/34 G01N21/77 G01N33/58 |
| X | CHRISTOPH GR?N: "O2-sensitive microcavity arrays: A new platform for oxygen measurements in 3D cell cultures", FRONTIERS IN BIOENGINEERING AND BIOTECHNOLOGY, Bd. 11, 20. Februar 2023 (2023-02-20), XP093153542, CH ISSN: 2296-4185, DOI: 10.3389/fbioe.2023.1111316 * Abstrakt, Material und Methoden, Abbildungen * ----- | 1-15 | |
| A | BRIGITTE ALTMANN: "Advanced 3D Cell Culture Techniques in Micro-Bioreactors, Part II: Systems and Applications", PROCESSES, Bd. 9, Nr. 1, 23. Dezember 2020 (2020-12-23), Seite 21, XP093153550, CH ISSN: 2227-9717, DOI: 10.3390/pr9010021 ----- | 1-15 | |

**RECHERCHIERTE SACHGEBIETE (IPC)**

G01N
C12M

-/--

Der vorliegende Recherchenbericht wurde für alle Patentansprüche erstellt

| Recherchenort | Abschlußdatum der Recherche | Prüfer |
|---|---|---|
| München | 15. Mai 2024 | Behrens, Ralf |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

......................................................................................
& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

**Nummer der Anmeldung**

EP 23 21 6372

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | VAN DER SANDEN BOUDEWIJN ET AL: "3D two-photon polymerization of smart cell gelatin - collagen matrixes with incorporated ruthenium complexes for the monitoring of local oxygen tensions", ACTA BIOMATERIALIA, ELSEVIER, AMSTERDAM, NL, Bd. 130, 12. Juni 2021 (2021-06-12), Seiten 172-182, XP086699438, ISSN: 1742-7061, DOI: 10.1016/J.ACTBIO.2021.06.021 [gefunden am 2021-06-12] ----- | 1-15 | |
| X | ERIC GOTTWALD: "O2-sensitive Mikrokavit?tenarrays: 3D-Zellkultursystem mit Sensorfunktion", BIOSPEKTRUM, Bd. 29, Nr. 7, 1. November 2023 (2023-11-01), Seiten 758-761, XP093153760, DE ISSN: 0947-0867, DOI: 10.1007/s12268-023-2045-1 Gefunden im Internet: URL:https://link.springer.com/article/10.1007/s12268-023-2045-1/fulltext.html> * Abbildung 1, Seite 760 * ----- | 1-15 | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| München | 15. Mai 2024 | Behrens, Ralf |

EPO FORM 1503 03.82 (P04C03)

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 23180517

- EP 3765599 B1

**In der Beschreibung aufgeführte Nicht-Patentliteratur**

- **WANG, X.D** ; **O.S. WOLFBEIS**. Optical methods for sensing and imaging oxygen: materials, spectroscopies and applications. *Chem. Soc. Rev*, 2014, vol. 43 (10), 3666-761

- **ONAKPOYA, I. J** ; **HENEGHAN, C. J** ; **ARONSON, J. K**. Post-marketing withdrawal of 462 medicinal products because of adverse drug reactions: a systematic review of the world literature. *BMC Med.*, 2016, vol. 14 (10)

- **WENGER, R. H.** ; **KURTCUOGLU, V** ; **SCHOLZ, C. C** ; **MART, H. H** ; **HOOGEWIJS, D**. Frequently asked questions in hypoxia research. *Hypoxia*, 2015, vol. 3, 35-43

- **DRAKHLIS, L** ; **S. BISWANATH** ; **C. M. FARR** ; **V. LUPANOW** ; **J. TESKE** ; **K. RITZENHOFF** ; **A. FRANKE** ; **F. MANSTEIN,** ; **E. BOLESANI** ; **H. KEMPF**. Human heart-forming organoids recapitulate early heart and foregut development. *Nat Biotechnol.*, 2021

- **DRAKHLIS** ; **LIKA** ; **SANTOSHI BISWANATH DEVADAS** ; **ROBERT ZWEIGERDT**. Generation of heart-forming organoids from human pluripotent stem cells. *Nature Protocols*, 2021, vol. 16, 5652-72

- **HALLOIN, CAROLINE** ; **KRISTIN SCHWANKE,** ; **WIEBKE LÖBEL** ; **ANNIKA FRANKE** ; **MONIKA SZEPES** ; **SANTOSHI BISWANATH** ; **STEPHANIE WUNDERLICH** ; **SYLVIA MERKERT** ; **NATALIE WEBER,** ; **FELIX OSTEN**. Continuous WNT Control Enables Advanced hPSC Cardiac Processing and Prognostic Surface Marker Identification in Chemically Defined Suspension Culture. *Stem Cell Reports*, 2019, vol. 13, 366-79

- **RICCI SIGNORINI, M. E.** ; **M. SZEPES** ; **A. MELCHERT** ; **M. BAKAR** ; **S. MERKERT** ; **A. HAASE,** ; **G. GOHRING** ; **U. MARTIN** ; **I. GRUH**. Generation of human induced pluripotent stem cell lines encoding for genetically encoded calcium indicators RCaMP1h and GCaMP6f. *Stem Cell Res,*, 2022, vol. 60, 102697

- **CHRISTOPH GRÜN** ; **JANA PFEIFER,** ; **GREGOR LIEBSCH** ; **ERIC GOTTWALD**. O2-sensitive microcavity arrays: A new platform for oxygen measurements in 3D cell cultures. *Frontiers in Bioengineering and Biotechnology*, 2023